(19) Europäisches Patentamt
European Patent Office
Office européen des brevets

(11) EP 3 389 660 B1

(12) **EUROPEAN PATENT SPECIFICATION**

(45) Date of publication and mention of the grant of the patent:
**16.10.2019 Bulletin 2019/42**

(21) Application number: **16816563.7**

(22) Date of filing: **08.12.2016**

(51) Int Cl.:
*A61K 31/4745* (2006.01) *A61K 31/497* (2006.01)
*A61P 35/00* (2006.01) *A61K 9/00* (2006.01)

(86) International application number:
**PCT/US2016/065503**

(87) International publication number:
**WO 2017/105982 (22.06.2017 Gazette 2017/25)**

(54) **COMBINATION THERAPY FOR CANCER**
KOMBINATIONSTHERAPIE GEGEN KREBS
POLYTHÉRAPIE CONTRE LE CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.12.2015 US 201562267462 P**

(43) Date of publication of application:
**24.10.2018 Bulletin 2018/43**

(73) Proprietor: **Eli Lilly and Company**
**Indianapolis, IN 46285 (US)**

(72) Inventors:
- **BECKMANN, Richard Paul**
**Indianapolis, IN 46206-6288 (US)**
- **DONOHO, Gregory Paul**
**Indianapolis, IN 46206-6288 (US)**
- **LIN, Aimee Karis**
**Indianapolis, IN 46206-6288 (US)**
- **WACHECK, Volker**
**Indianapolis, IN 46206-6288 (US)**

(74) Representative: **Smith, Andrew George**
**Eli Lilly and Company Limited**
**European Patent Operations**
**Lilly Research Centre**
**Erl Wood Manor**
**Windlesham, Surrey GU20 6PH (GB)**

(56) References cited:
**WO-A1-2010/077758 WO-A1-2012/097039**

- **MASSEY ANDREW J ET AL: "mTORC1 and DNA-PKcs as novel molecular determinants of sensitivity to Chk1 inhibition", MOLECULAR ONCOLOGY, vol. 10, no. 1, 25 August 2015 (2015-08-25), pages 101-112, XP029362559, ISSN: 1574-7891, DOI: 10.1016/J.MOLONC.2015.08.004**
- **JOGITHA SELVARAJAH ET AL: "DNA damage-induced S and G2/M cell cycle arrest requires mTORC2-dependent regulation of Chk1", ONCOTARGET, vol. 1, no. 1, 15 November 2014 (2014-11-15), pages 427-440, XP055345105, DOI: 10.18632/oncotarget.2813**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 389 660 B1

## Description

The present invention relates to a combination of 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, with 5-(5-(2-(3-aminopropoxy)-6-methoxyphenyl)-1H-pyrazol-3-ylamino) pyrazine-2-carbonitrile, or a pharmaceutically acceptable salt thereof, and to methods of using the combination to treat certain disorders, such as cancer, in particular squamous histology cancers, breast cancer, prostate cancer, bladder cancer, cervical cancer, endometrial cancer, ovarian cancer, and colorectal cancer including, most particularly, squamous non-small cell lung cancer (squamous NSCLC), head and neck squamous cell carcinoma (HNSCC), ovarian cancer, esophageal cancer, anal cancer, and triple negative breast cancer (TNBC).

Checkpoint kinase 1 (CHK1) plays a key role in the homologous recombination repair (HRR) pathway, which repairs double strand breaks (DSB), including DSB that may be caused by CHK1 inhibition. Phosphoinositide 3-kinase (PI3 kinase or PI3K) also is involved in HRR. PI3K Class IA isoforms are sensors of genomic instability and regulate the Nbs1 sensor protein and Rad51 foci formation (Juvekar et al., Cancer Discov. 2012;2(11):1048-1063; Kumar et al., PNAS USA. 2011;107:7491-7496). Additionally, inhibition of PI3K results in homologous recombination deficiency in preclinical models of TNBC (estrogen receptor negative (ER-), progesterone receptor negative (PR-) and human epidermal growth factor receptor 2 negative (HER-2-)) with PI3K-activating alterations by down regulating BRCA 1 and BRCA2 (Ibrahim et al., Cancer Discov. 2012;2(11):1036-1047). Furthermore, regulation of CHK1 by the mammalian target of rapamycin (mTOR) pathway has been linked to DNA replication in response to replication stress (Shen et al., Cancer Res. 2012;72(8)Supp.1.Abst.2535). As a result, it is hypothesized that inhibition of PI3K and CHK1 may disrupt HRR and the repair of DSB caused by CHK1 inhibition and that inhibition of mTOR and CHK1 may impact the response to replication stress, which may result in enhanced antitumor activity and lead to improved outcomes for patients.

Broadly applicable therapies for cancer, in particular squamous histology cancers, breast cancer, prostate cancer, bladder cancer, cervical cancer, endometrial cancer, ovarian cancer, and colorectal cancer including, most particularly, squamous NSCLC, HNSCC, ovarian cancer, esophageal cancer, anal cancer, and TNBC still remain elusive and, thus, there exists a need for more and different therapies that may prove to be effective in treating one or more of these cancers.

8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one is a dual inhibitor of PI3K and mTOR kinase. The compound and methods of making and using this compound including for the treatment of cancer and more specifically for the treatment of colon cancer, head and neck cancer, NSCLC, breast cancer, ovarian cancer, prostate cancer, and endometrial cancer are disclosed in WO 2012/097039. Furthermore, this compound is being investigated in clinical trials for advanced/metastatic cancer (including lymphoma), mesothelioma (as monotherapy or in combination with pemetrexed/cisplatin), breast cancer (in combination with fulvestrant), recurrent or persistent endometrial cancer, as well as in squamous non-small cell lung cancer (as monotherapy or in combination with necitumumab), and metastatic castration resistant prostate cancer (in combination with enzalutamide).

5-(5-(2-(3-Aminopropoxy)-6-methoxyphenyl)-1H-pyrazol-3-ylamino) pyrazine-2-carbonitrile is a CHK1 inhibitor and, to a lesser extent, a CHK2 inhibitor. The compound and methods of making and using this compound including for the treatment of cancer and more specifically for the treatment of colon cancer, lung cancer, mammary cancer, ovarian cancer, and uterine cancer are disclosed in WO 2010/077758. The 5-(5-(2-(3-aminopropoxy)-6-methoxyphenyl)-1H-pyrazol-3-ylamino)pyrazine-2-carbonitrile compound can be used as the methanesulfonate hydrate form, i.e. 5-(5-(2-(3-aminopropoxy)-6-methoxyphenyl)-1H-pyrazol-3-ylamino)pyrazine-2-carbonitrile methanesulfonate hydrate. Alternative compound names for this form include 2-pyrazinecarbonitrile, 5-[[5-[2-(3-aminopropyl)-6-methoxyphenyl]-1H-pyrazol-3-yl]amino] monomesylate monohydrate (see WO 2010/077758). Additionally, the 5-(5-(2-(3-aminopropoxy)-6-methoxyphenyl)-1H-pyrazol-3-ylamino)pyrazine-2-carbonitrile compound can be used as the lactate monohydrate form, i.e. 5-(5-(2-(3-aminopropoxy)-6-methoxyphenyl)-1H-pyrazol-3-ylamino)pyrazine-2-carbonitrile (S)-lactate monohydrate, as detailed herein below. Furthermore, this compound is being investigated in clinical trials for advanced cancer, breast cancer, ovarian cancer, squamous cell carcinoma, anal cancer, NSCLC, small cell lung cancer, and HNSCC as well as in colorectal neoplasms (in combination with cetuximab) and head and neck neoplasms (in combination with cisplatin/radiation or cetuximab/radiation).

Novel methods of using the combination of 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, and 5-(5-(2-(3-aminopropoxy)-6-methoxyphenyl)-1H-pyrazol-3-ylamino)pyrazine-2-carbonitrile, or a pharmaceutically acceptable salt thereof, to treat cancer, in particular squamous histology cancers, breast cancer, prostate cancer, bladder cancer, cervical cancer, endometrial cancer, ovarian cancer, and colorectal cancers including, most particularly, ovarian cancer, squamous NSCLC, HNSCC, esophageal cancer, anal cancer, and TNBC are herein presented.

Certain combinations of CHK1 inhibitors and PI3 kinase and/or mTOR inhibitors have been contemplated in the art. More particularly, disclosures include certain combinations of mTOR inhibitors AZD8055, RAD-001, rapamycin,

and BEZ235 inducing synergistic cytotoxicity with the CHK1 inhibitor V158411 in p53 mutant colon cancer cells (Massey et al., Molecular Oncology (2015) 1-12) and of methods of treating a subject having a LBK1-null cancer by administering to the subject a compound that inhibits the expression of activity of deoxythymidlate kinase (DTYMK), checkpoint kinase 1 (CHK1) or both wherein CHK1 inhibitors include for example LY2606368 and optionally the subject is further administered a chemotherapeutic agent such as a tyrosine kinase inhibitor or an mTOR inhibitor (WO2013/103836).

Accordingly, the present invention provides a compound of the formula:

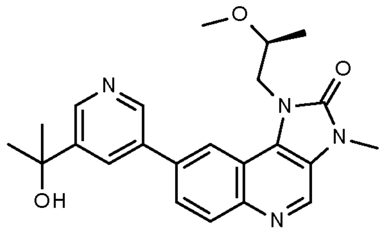

,

or a pharmaceutically acceptable salt thereof, for simultaneous, separate, or sequential use in combination with a compound of the formula:

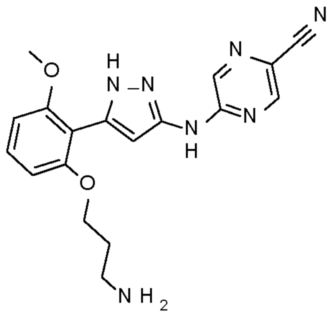

,

or a pharmaceutically acceptable salt thereof in the treatment of squamous histology cancers, breast cancer, prostate cancer, bladder cancer, cervical cancer, endometrial cancer, ovarian cancer, and colorectal cancer.

The present invention also provides a compound of the formula:

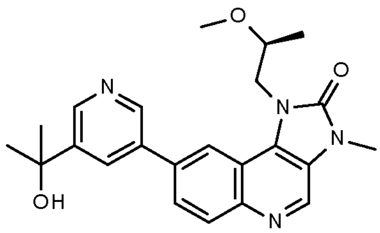

,

or a pharmaceutically acceptable salt thereof, for simultaneous, separate, or sequential use in combination with a compound of formula:

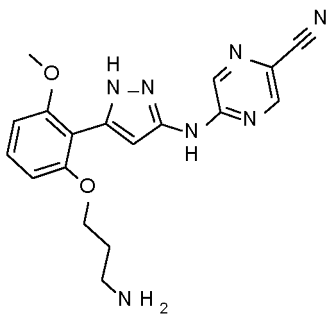

,

or a pharmaceutically acceptable salt thereof in the treatment of squamous histology cancers, breast cancer, prostate cancer, bladder cancer, cervical cancer, endometrial cancer, ovarian cancer, and colorectal cancer., wherein

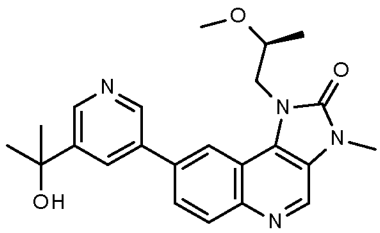

,

or a pharmaceutically acceptable salt thereof, is administered at a dose of about 100 mg twice per day to about 200 mg twice per day on Days 1 to 14 of a 14-day cycle and

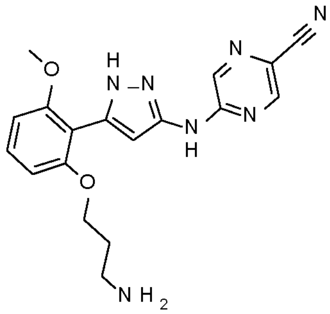

,

or a pharmaceutically acceptable salt thereof, is administered at a dose of about 60 $mg/m^2$ to about 105 $mg/m^2$ on Day 1 of a 14-day cycle or every 14 or 21 days. Additionally, the present invention provides

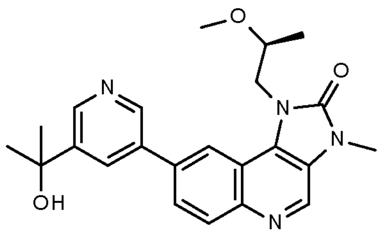

,

or a pharmaceutically acceptable salt thereof, administered at a dose of about 100 mg twice per day on Days 1 to 14 of a 14-day cycle and

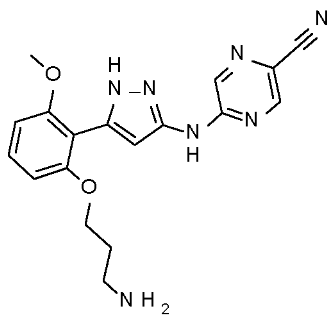

,

or a pharmaceutically acceptable salt thereof, is administered at a dose of about 60 $mg/m^2$ on Day 1 of a 14-day cycle or every 14 or 21 days. Furthermore, the present invention provides

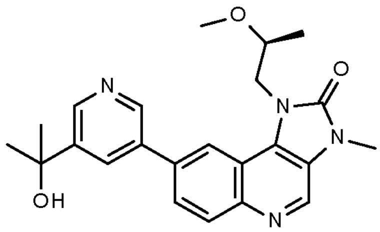

,

or pharmaceutically acceptable salt thereof, administered at a dose of about 100 mg twice per day on Days 1 to 14 of a 14-day cycle and

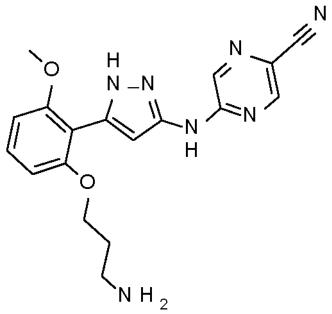

,

or a pharmaceutically acceptable salt thereof, is administered at a dose of about 105 $mg/m^2$ on Day 1 of a 14-day cycle or every 14 or 21 days. In addition, the present invention provides

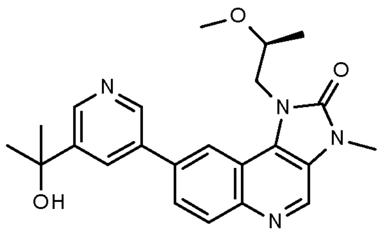

,

or pharmaceutically acceptable salt thereof, administered at a dose of about 200 mg twice per day on Days 1 to 14 of a 14-day cycle and

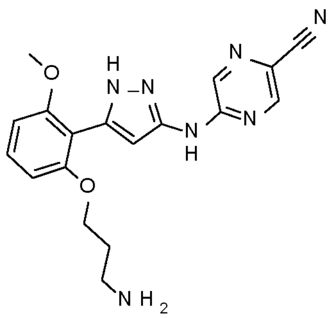

,

or a pharmaceutically acceptable salt thereof, is administered at a dose of about 105 $mg/m^2$ on Day 1 of a 14-day cycle or every 14 or 21 days.

The present invention also provides

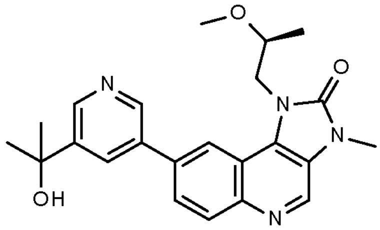

,

or a pharmaceutically acceptable salt thereof, administered orally and

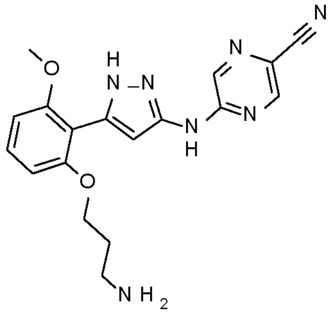

,

or a pharmaceutically acceptable salt thereof, administered by intravenous infusion.

In addition, the invention provides a kit comprising a compound of the formula:

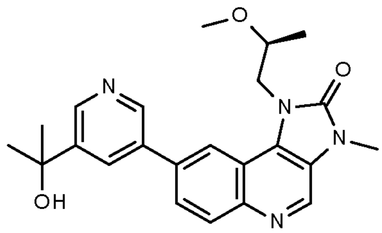

,

or a pharmaceutically acceptable salt thereof, and a compound of the formula:

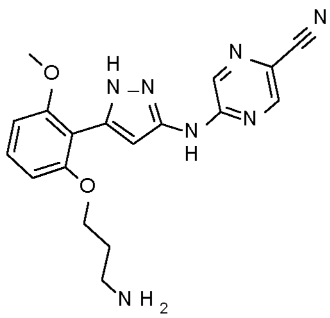

,

or a pharmaceutically acceptable salt thereof, for simultaneous, separate, or sequential use in the treatment of squamous histology cancers, breast cancer, prostate cancer, bladder cancer, cervical cancer, endometrial cancer, ovarian cancer, and colorectal cancer including, most particularly, squamous NSCLC, HNSCC, esophageal cancer, anal cancer, and TNBC.

The invention further provides a kit, comprising a pharmaceutical composition, comprising a compound of the formula:

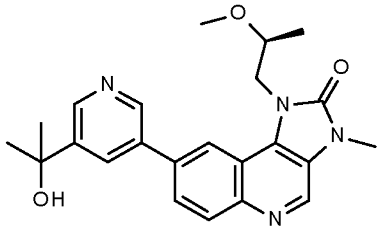

,

or a pharmaceutically acceptable salt thereof, with one or more pharmaceutically acceptable carriers, diluents, or excipients, and a pharmaceutical composition, comprising a compound of the formula:

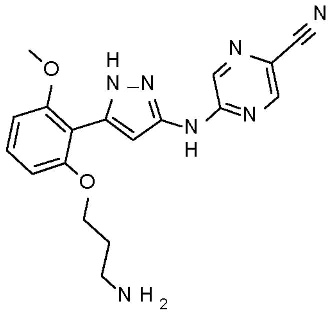

,

or a pharmaceutically acceptable salt thereof, with one or more pharmaceutically acceptable carriers, diluents, or excipients for simultaneous, separate, or sequential use in the treatment of squamous histology cancers, breast cancer, prostate cancer, bladder cancer, cervical cancer, endometrial cancer, ovarian cancer, and colorectal cancer including, most particularly, squamous NSCLC, HNSCC, esophageal cancer, anal cancer, and TNBC.

Figure 1 represents the % response of anti-tumor effects of 5-(5-(2-(3-aminopropoxy)-6-methoxyphenyl)-1H-pyrazol-3-ylamino)pyrazine-2-carbonitrile methanesulfonate hydrate in PDX mouse models of TNBC.
Figure 2 represents the % response of anti-tumor effects of 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one in PDX mouse models of TNBC.
Figure 3 represents the % response of anti-tumor effects of 5-(5-(2-(3-aminopropoxy)-6-methoxyphenyl)-1H-pyrazol-3-ylamino)pyrazine-2-carbonitrile methanesulfonate hydrate and 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one in PDX mouse models of TNBC.

As used herein, the compound name "8-[5-(1-hydroxy-1-methylethyl)pyridin-3-yl]-1-[(2S)-2-methoxypropyl]-3-methyl-1,3-dihydro-2H-imidazo[4,5-c]quinolin-2-one" is disclosed in WO 2012/097039 and refers to the compound with the following structure:

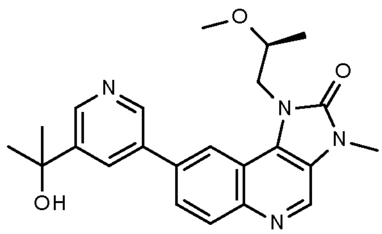

.

The CAS registry number for this compound is 1386874-06-1. Alternative compound names include 2*H*-Imidazo[4,5-c]quinolin-2-one, 1,3-dihydro-8-[5-(1-hydroxy-1-methylethyl)-3-pyridinyl]-1-[(2*S*)-2-methoxypropyl]-3-methyl-.

As used herein, the compound name "5-(5-(2-(3-aminopropoxy)-6-methoxyphenyl)-1H-pyrazol-3-ylamino)pyrazine-2-carbonitrile" is disclosed in WO 2010/077758 and refers to the compound with the following structure:

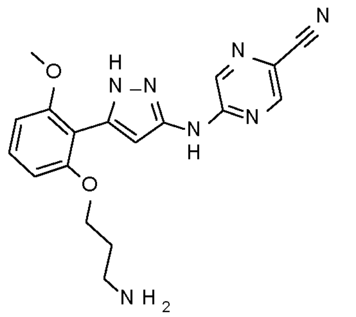

The CAS registry number for this compound is 1234015-52-1. Alternative compound names include 2-pyrazinecarbonitrile, 5-[[5-[2-(3-aminopropoxy)-6-methoxyphenyl]-1H-pyrazo1-3-yl]amino]-.

As used herein, the compound named 2-pyrazinecarbonitrile, 5-[[5-[2-(3-aminopropyl)-6-methoxyphenyl]-1H-pyrazol-3-yl]amino] monomesylate monohydrate is disclosed in WO 2010/077758 and refers to the compound with the following structure:

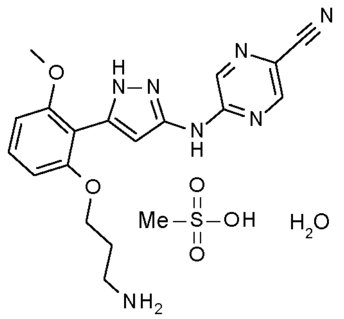

The CAS registry number for this compound is 1234015-57-6. Alternative compound names include 5-(5-(2-(3-aminopropoxy)-6-methoxyphenyl)-1H-pyrazol-3-ylamino)pyrazine-2-carbonitrile methanesulfonate hydrate.

As used herein, the compound named 5-(5-(2-(3-aminopropoxy)-6-methoxyphenyl)-1H-pyrazol1-3-ylamino)pyrazine-2-carbonitrile (S)-lactate monohydrate refers to the compound with the following structure:

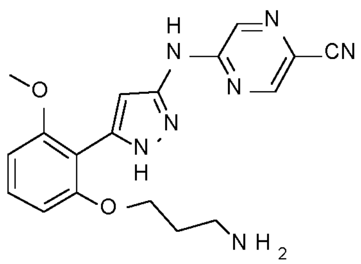

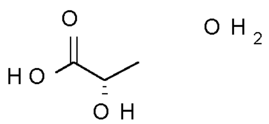

Details relevant to 5-(5-(2-(3-aminopropoxy)-6-methoxyphenyl)-1H-pyrazol-3-ylamino)pyrazine-2-carbonitrile (S)-lactate monohydrate preparation, crystalline form, formulation, and preparation of IV (intravenous) injection are provided herein below.

Batch Processing

Scheme 1

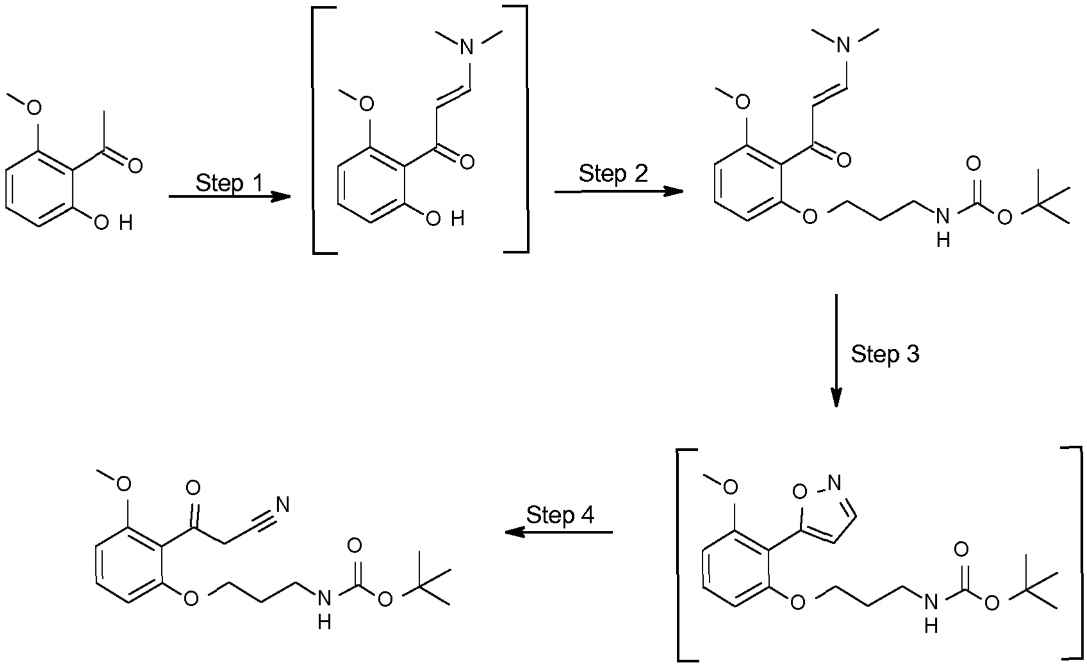


Continuous Flow Processing

Scheme 2

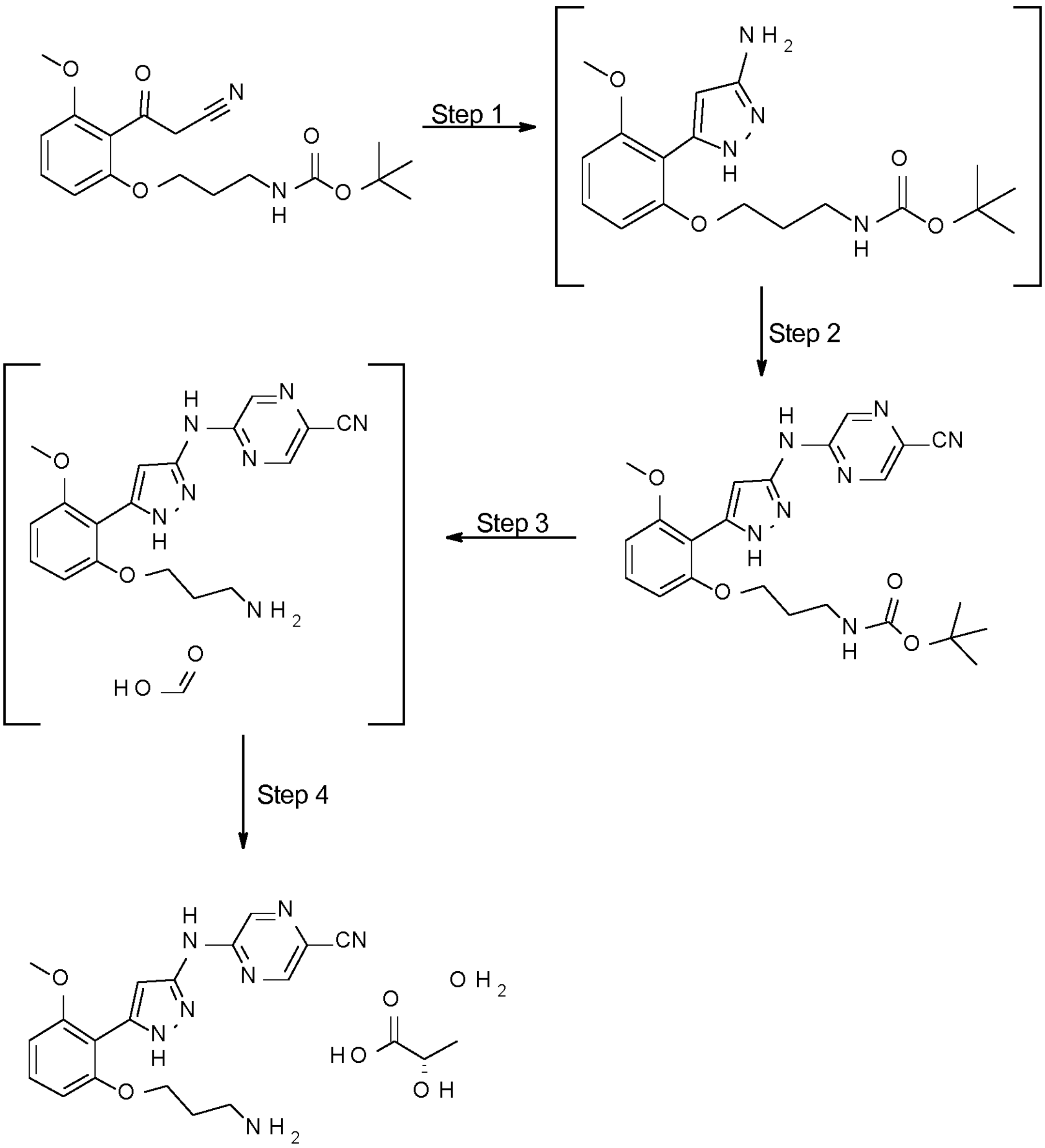


Preparation 1

tert-Butyl (E)-(3-(2-(3-(dimethylamino)acryloyl)-3-methoxyphenoxy)propyl)carbamate

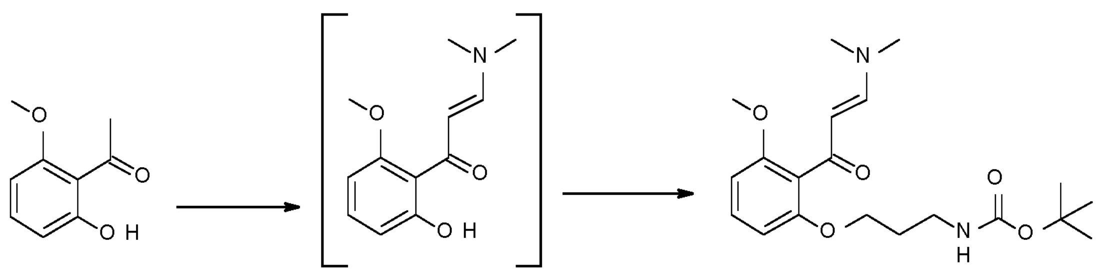

Combine 1-(2-hydroxy-6-methoxyphenyl)ethan-1-one (79.6 kg, 479 mol) and 1,1-dimethoxy-N,N-dimethylmethanamino (71.7 kg, 603.54 mol) with DMF (126 kg). Heat to 85-90 °C for 12 hours. Cool the reaction mixture containing intermediate (E)-3-(dimethylamino)-1-(2-hydroxy-6-methoxyphenyl)prop-2-en-1-one (mp 84.74 °C) to ambient temperature and add anhydrous potassium phosphate (136 kg, 637.07 mol) and tert-butyl (3-bromopropyl)carbamate (145 kg, 608.33 mol). Stir the reaction for 15 hours at ambient temperature. Filter the mixture and wash the filter cake with MTBE (3×, 433 kg, 300 kg, and 350 kg). Add water (136 kg) and aqueous sodium chloride (25% solution, 552 kg) to the combined MTBE organic solutions. Separate the organic and aqueous phases. Back-extract the resulting aqueous phase with MTBE (309 kg) and add the MTBE layer to the organic solution. Add an aqueous sodium chloride solution (25% solution, 660 kg) to the combined organic extracts and separate the layers. Concentrate the organic extracts to 1,040 kg - 1,200 kg and add water (400 kg) at 30-35 °C to the residue. Cool to ambient temperature and collect material by filtration as a wet cake to give the title product (228.35 kg, 90%). ES/MS (m/z): 379.22275 (M+1).

Preparation 2

tert-Butyl (3 -(2-(2-cyanoacetyl)-3 -methoxyphenoxy)propyl)carbamate

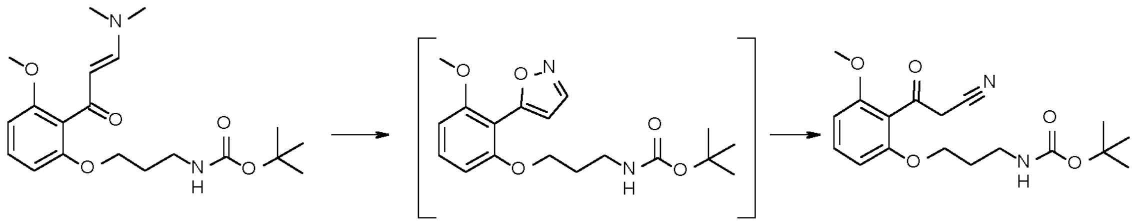

Combine ethanol (1044 kg), hydroxyl amino hydrochloride (30 kg, 431.7 mol), and tert-butyl (E)-(3-(2-(3-(dimethylamino)acryloyl)-3-methoxyphenoxy)propyl)carbamate (228.35 kg, 72% as a wet water solid, 434.9 mol) to form a solution. Heat the solution to 35 - 40 °C for 4-6 hours. Cool the reaction to ambient temperature and concentrate to a residue. Add MTBE (300 kg) to the residue and concentrate the solution to 160 kg - 240 kg. Add MTBE (270 kg) and concentrate the solution. Add MTBE (630 kg), water (358 kg), and sodium chloride solution (80 kg, 25% aqueous) and stir for 20 minutes at ambient temperature. Let the mixture stand for 30 minutes. Separate the aqueous layer. Add water (360 kg) and sodium chloride solution (82 kg, 25% sodium chloride) to the organic phase. Stir for 20 minutes at ambient temperature. Let the mixture stand for 30 minutes. Separate the aqueous portion. Add sodium chloride solution (400 kg, 25 % aqueous) to the organic portion. Stir for 20 minutes at ambient temperature. Let the mixture stand for 30 minutes at ambient temperature. Separate the aqueous portion. Concentrate the organic portion to 160 kg - 240 kg at 40 °C. Add ethanol (296 kg) to the organic portion. Concentrate the solution to 160 kg to 240 kg at 40 °C to provide an intermediate of tert-butyl (3-(2-(isoxazol-5-yl)-3-methoxyphenoxy)propyl)carbamate. Add ethanol (143 kg) and water (160 kg) to the concentrated solution. Add potassium hydroxide (31.8 kg) at 40 °C. Add ethanol (80 kg) and adjust the temperature to 45-50 °C. Stir for 4-6 hours at 45-50 °C and concentrate to 160 kg - 240 kg at 40 °C. Add water to the concentrate (160 kg) and acetic acid (9.0 kg) drop-wise to adjust the pH to 10-12 while maintaining the temperature of the solution at 25 to 35 °C. Add ethyl acetate (771 kg) and acetic acid drop-wise to adjust the pH to 5-7 while maintaining the temperature of the solution at 25-35 °C. Add sodium chloride solution (118 kg, 25% aqueous solution). Stir the mixture for 20 minutes at ambient temperature. Let the solution stand for 30 minutes at ambient temperature. Separate the aqueous portion. Heat the organic portion to 30-35 °C. Add water (358 kg) drop-wise. Stir the solution for 20 minutes while maintaining the temperature at 30 to 35 °C. Let the mixture stand for 30 minutes and separate the aqueous portion. Wash the organic portion with sodium chloride solution (588 kg, 25% aqueous) and concentrate the organic portion to 400 kg - 480 kg at 40-50 °C. Heat the concentrated solution to 50 °C to form a solution. Maintain the solution at 50 °C and add *n*-heptane (469 kg) drop-wise. Stir the solution for 3 hours at 50 °C before slowly cooling to ambient temperature to crystallize the product. Stir at ambient temperature for 15 hours and filter the crystals. Wash the crystals with ethanol/*n*-heptane (1:2, 250 kg) and dry at 45 °C for 24 hours to provide the title compound (133.4 kg, 79.9%), m.p. 104.22 °C.

Example 1

5-(5-(2-(3-Aminopropoxy)-6-methoxyphenyl)-1H-pyrazol-3-ylamino)pyrazine-2-carbonitrile (S)-lactate monohydrate

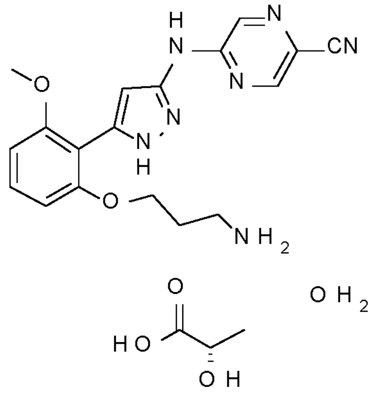

Combine a THF solution (22%) of tert-butyl (3-(2-(2-cyanoacetyl)-3-methoxyphenoxy)propyl)carbamate (1.0 eqv, this is define as one volume) with hydrazine (35%, 1.5 eqv), acetic acid (glacial, 1.0 eqv), water (1 volume based on the THF solution) and methanol (2 volumes based on the THF solution). As this is a continuous operation, grams or kg is irrelevant in this processing methodology. Heat the resulting mixture to 130 °C and 1379 kPa with a rate of V/Q = 70 minutes (where V refers to the volume of the reactor and Q refers to flow rate), tau = 60. Extract the solution with toluene (4 volumes), water (1 volume), and sodium carbonate (10% aqueous, 1 eqv). Isolate the toluene layer and add to DMSO (0.5 volumes). Collect a solution of the intermediate compound *tert*-butyl (3 -(2-(3 -amino-1H-pyrazol-5 -yl)-3 -methoxyphenoxy) propyl)carbamate (26.59 kg, 91%) in 10 days, mp = 247.17 °C as a DMSO solution (3 volumes of product). N-ethylmorpholine (1.2 eqv) and 5-chloropyrazine-2-carbonitrile (1.15 eqv) in 2 volumes of DMSO is combined in a tube reactor at 80 °C, V/Q = 3 and tau = 170 minutes at ambient pressure. Add the product stream to methanol (20 vol). As a continuous process, filter the mixture and wash with methanol followed by MTBE. Air dry the material on the filter to give tert-butyl (3-(2-(3-((5-cyanopyrazin-2-yl)amino)-1H-pyrazol-5-yl)-3-methoxyphenoxy) propyl)carbamate in a continuous fashion (22.2 kg, 88.7%, 8 days). Dissolve a solution of tert-butyl (3-(2-(3-((5-cyanopyrazin-2-yl)amino)-1H-pyrazol-5-yl)-3-methoxyphenoxy) propyl)carbamate in formic acid (99%, 142 kg) at ambient temperature and agitate for 4 hours to provide an intermediate of 5-((5-(2-(3-aminopropoxy)-6-methoxyphenyl)-1H-pyrazol-3-yl)amino)pyrazine-2-carbonitrile formate. Dilute the solution with water (55 kg), (S)-lactic acid (30%, 176 kg) and distill the resulting mixture until < 22 kg formic acid remains. Crystallize the resulting residue from THF and wash with a THF -water (0.5% in THF) solution. Dry the wet cake at 30 °C at >10% relative humidity to give the title product as a white to yellow solid (24.04 kg, 85-90%), m.p. 157 °C.

Alternate Preparation Example 1

5-(5-(2-(3-Aminopropoxy)-6-methoxyphenyl)-1H-pyrazol-3-ylamino)pyrazine-2-carbonitrile (S)-lactate monohydrate

Add 5-({3-[2-(3-aminopropoxy)-6-methoxyphenyl]-1H-pyrazol-5-yl}amino)pyrazine-2-carbonitrile (4.984 g, 13.33 mmol, 97.7 wt%) to *n*-PrOH (15.41 g, 19.21 mL) to form a slurry. Heat the slurry to 60 °C. Add (S)-lactic acid (1.329 g, 14.75 mmol) to water (19.744 mL) and add this solution to the slurry at 58 °C. Heat the solution to 60 °C and add *n*-PrOH (21.07 g, 26.27 mL). Seed the solution with 5-((5-(2-(3 -aminopropoxy)-6-methoxyphenyl)-1H-pyrazo 1-3 -yl)amino)pyrazine-2-carbonitrile (S)-lactate monohydrate (48.8 mg, 0.1 mmol) and cool the solution to 40 °C over 35 minutes. Add *n*-PrOH (60.5 mL) to the slurry at 40 °C via a syringe pump over 2 hours and maintain the temperature at 40 °C. Once complete, air cool the slurry to ambient temperature for 2 hours, then cool the mixture in ice-water for 2 hours. Filter the product, wash the wet cake with 6:1 (v/v) *n*-PrOH : $H_2O$ (15 mL), followed by *n*-PrOH (15 mL) and dry the wet cake for 20 minutes. Dry the solid overnight at 40 °C in vacuo to give the title compound as a white to yellow solid (5.621 g, 89.1%), m.p. 157 °C.

Crystalline Example 1

Crystalline 5-(5-(2-(3-aminopropoxy)-6-methoxyphenyl)-1H-pyrazol-3-ylamino)pyrazine-2-carbonitrile (S)-lactate monohydrate

Prepare a slurry having 5-(5-(2-(3-aminopropoxy)-6-methoxyphenyl)-1H-pyrazol-3-ylamino)pyrazine-2-carbonitrile (368 mg, 1.0 mmol) in a 10:1 THF-water (5 mL) solution and stir at 55 °C. Add (S)-lactic acid (110 mg, 1.22 mmol) dissolved in THF (1 mL). A clear solution forms. Stir for one hour. Reduce the temperature to 44 °C and stir until an off-white precipitate forms. Filter the material under vacuum, rinse with THF, and air dry to give the title compound

(296 mg, 80%).

X-Ray Powder Diffraction, Crystalline Example 1

Obtain the XRPD (X-Ray Powder Diffraction) patterns of the crystalline solids on a Bruker D4 Endeavor X-ray powder diffractometer, equipped with a CuKa source ($\lambda$ = 1.54060 Å) and a Vantec detector, operating at 35 kV and 50 mA. Scan the sample between 4 and 40° in 2$\theta$, with a step size of 0.0087° in 2$\theta$ and a scan rate of 0.5 seconds/step, and with 0.6 mm divergence, 5.28mm fixed anti-scatter, and 9.5 mm detector slits. Pack the dry powder on a quartz sample holder and obtain a smooth surface using a glass slide. It is well known in the crystallography art that, for any given crystal form, the relative intensities of the diffraction peaks may vary due to preferred orientation resulting from factors such as crystal morphology and habit. Where the effects of preferred orientation are present, peak intensities are altered, but the characteristic peak positions of the polymorph are unchanged. See, *e.g.* The U. S. Pharmacopeia 35 - National Formulary 30 Chapter <941> Characterization of crystalline and partially crystalline solids by X-ray powder diffraction (XRPD) Official December 1, 2012-May 1, 2013. Furthermore, it is also well known in the crystallography art that for any given crystal form the angular peak positions may vary slightly. For example, peak positions can shift due to a variation in the temperature or humidity at which a sample is analyzed, sample displacement, or the presence or absence of an internal standard. In the present case, a peak position variability of $\pm$ 0.2 in 2$\theta$ will take into account these potential variations without hindering the unequivocal identification of the indicated crystal form. Confirmation of a crystal form may be made based on any unique combination of distinguishing peaks (in units of ° 2$\theta$), typically the more prominent peaks. The crystal form diffraction patterns, collected at ambient temperature and relative humidity, were adjusted based on NIST 675 standard peaks at 8.85 and 26.77 degrees 2-theta.

Characterize a prepared sample of crystalline 5-(5-(2-(3-aminopropoxy)-6-methoxyphenyl)-1H-pyrazol-3-ylamino)pyrazine-2-carbonitrile (S)-lactate monohydrate by an XRPD pattern using CuKa radiation as having diffraction peaks (2-theta values) as described in Table 1 below. Specifically the pattern contains a peak at 12.6 in combination with one or more of the peaks selected from the group consisting of 24.8, 25.5, 8.1, 6.6, 12.3, and 16.3 with a tolerance for the diffraction angles of 0.2 degrees.

X-ray powder diffraction peaks of Crystalline Example 1

Table 1

| Peak | Angle (2-Theta) +/-0.2° | Relative Intensity (% of most intense peak) |
|---|---|---|
| 1 | 6.6 | 58 |
| 2 | 8.1 | 63 |
| 3 | 12.3 | 61 |
| 4 | 12.6 | 100 |
| 5 | 16.3 | 57 |
| 6 | 20.9 | 34 |
| 7 | 21.1 | 49 |
| 8 | 24.8 | 87 |
| 9 | 25.1 | 31 |
| 10 | 25.5 | 78 |

Formulation of 5-(5-(2-(3-aminopropoxy)-6-methoxyphenyl)-1H-pyrazol-3-ylamino)pyrazine-2-carbonitrile (S)-lactate monohydrate

Add warm water (2500 mL, 35-40 °C) and 5-((5-(2-(3-aminopropoxy)-6-methoxyphenyl)-1H-pyrazol-3-yl)amino)pyrazine-2-carbonitrile (S) lactate monohydrate (22.208 g, 46.9 mmol) to a manufacturing vessel with stirring. Stir for 10 minutes until the material dissolves and yields a clear solution. Add trehalose dihydrate (26.28 g, 69.46 mmol), mannitol (146.92 g, 806.49 mmol), and polysorbate 80 (6.528 g, 4.98 mmol) to the manufacturing vessel. Stir until dissolved. Add water (788.29 mL) to bring the solution to the final batch weight (3288.29 mL). Filter the solution through

a sterile 0.22 micron PVDF (polyvinylidene fluoride) membrane (Millipore Durapore ®) into a clean, dry receiving vessel. Fill the drug product solution into sterile vials (20.6 mL fill into 50 mL vial; total vials: 144 including 5 thermocouple vials). Upon completion of filling, partially stopper vials and place into the lyophilizer. Upon completion of the lyophilization cycle (4 days), fully stopper the vials under a slight vacuum (662 mbar) and seal. Store all vials at room temperature (15-25 °C).

Alternate Formulation of 5-(5-(2-(3-aminopropoxy)-6-methoxyphenyl)-1H-pyrazol-3-ylamino)pyrazine-2-carbonitrile (S)-lactate monohydrate

Add Water for Injection, (WFI) at room temperature (4000 mL) to a manufacture vessel. Add polysorbate 80 (6.25 g, 4.77 mmol) with stirring. Stir until the material dissolves and yields a clear solution. Add mannitol (150.0 g, 823.4 mmol) and stir until visually clear. Add trehalose dihydrate (129.86 g, 343.2 mmol) and then stir until visually clear. Add 1 N lactic acid (3.25 mL per L of batch solution) to the manufacturing vessel and stir until visually clear. Add 5-((5-(2-(3-aminopropoxy)-6-methoxyphenyl)-1H-pyrazol-3-yl)amino)pyrazine-2-carbonitrile (S) lactate monohydrate (21.71 g, 45.8 mmol) to the manufacturing vessel and stir until dissolved. Add WFI to bring the solution to the final batch volume (5000 mL). Filter the solution through a sterile 0.22 micron PVDF membrane (Millipore Durapore ®) into a clean, dry receiving vessel. Fill the drug product solution into sterile vials (20.6 mL fill into 50 mL vial). Upon completion of filling, partially stopper the vials and place into the lyophilizer. Upon completion of the lyophilization cycle (3-4 days), fully stopper the vials under a slight vacuum (about 866 mbar) and seal. Store all vials at refrigerated conditions (2-8 °C).

Preparation of 5-(5-(2-(3-aminopropoxy)-6-methoxyphenyl)-1H-pyrazol-3-ylamino)pyrazine-2-carbonitrile (S)-lactate monohydrate salt for IV injection

Dispense Water for Injection (WFI) equivalent to approximately 80% of the final batch volume. Warm the WFI to 40 °C ± 3 °C and transfer to the manufacturing vessel. Weigh the required amount of active pharmaceutical ingredient and after ensuring that the WFI temperature is still within the specified range, quantitatively transfer the active pharmaceutical ingredient into the manufacturing vessel while stirring. Stir until the active pharmaceutical ingredient has dissolved yielding a clear yellow solution, stirring for no longer than 30 minutes. Add trehalose and polysorbate 80 to the manufacturing vessel. Stir until dissolved. Measure the pH of the solution and adjust to pH 4.4 ± 0.3 with lactic acid (Note: If using lactic acid 88%-92% solution, titrate slowly and cautiously as small quantities can produce large changes in pH, or prepare a 10% solution of lactic acid for pH adjustment). If necessary, and only if the pH drops below the lower limit during adjustment, 10% sodium hydroxide solution can be added to bring the pH back into range. Add WFI to the manufacturing vessel to bring the solution to the final batch weight which is calculated based on the desired batch volume and the density of the solution. The pH of the solution is measured and adjusted, if necessary, to pH 4.4 ± 0.3 with lactic acid or NaOH. The solution is filtered through a sterile 0.22 micron PVDF membrane (Millipore Durapore ®) into a sterile receiving vessel. Fill the drug product into sterile vials. Upon completion of filling, vials are stoppered and sealed. If necessary, store all vials at -20 °C. The unit formula is shown in Table 2.

Table 2

| Unit Formula Active Ingredient | Quantity (mg/mL) |
|---|---|
| (S)-lactate monohydrate[1] salt | 2.59 (Equivalent to 2.0 free base) |
| Other Ingredients[2] | |
| Trehalose | 14.74 |
| Polysorbate 80 | 0.76 |
| Water for Injection | q.s. |
| Lactic Acid or Sodium hydroxide solution3 | - - |

[1] The amount of active pharmaceutical ingredient may be adjusted to take into account the Assay "as is, free base" of the drug substance. Assay "as is, free base" can be defined as the portion of the drug substance (fraction or a percentage on a mass basis) that is comprised of the active moiety, as measured by an appropriate analytical chemistry technique and that is not corrected for the presence of volatile substances.
[2] A reasonable variation of +/-10% is allowed for each excipient unless otherwise stated.
[3] Quantity sufficient to adjust pH.

Alternate Preparation of 5-(5-(2-(3-aminopropoxy)-6-methoxyphenyl)-1H-pyrazol-3-ylamino)pyrazine-2-carbonitrile (S)-lactate monohydrate salt for IV injection

Dispense WFI equivalent to approximately 80% of the final batch volume. Weigh the required amount of polysorbate 80 and add to the vessel while stirring until visually clear. Weigh the required amount of mannitol and add to the vessel while stirring until visually clear. Weigh the required amount of trehalose dihydrate and add to the vessel while stirring until visually clear. Add the appropriate amount of lactic acid to the vessel while stirring until visually clear. Weigh the required amount of active pharmaceutical ingredient and transfer the active pharmaceutical ingredient into the manufacturing vessel while stirring. Stir until the active pharmaceutical ingredient has dissolved yielding a clear yellow solution. Measure the pH of the solution and adjust to pH 4.2 ± 0.3 with lactic acid (Note: If using lactic acid 88%-92% solution, titrate slowly and cautiously as small quantities can produce large changes in pH, or prepare a 10% solution of lactic acid for pH adjustment). Add WFI to the manufacturing vessel to bring the solution to the final batch weight which is calculated based on the desired batch volume and the density of the solution. The pH of the solution is measured and adjusted, if necessary, to pH 4.2 ± 0.3 with lactic acid. The solution is filtered through a sterile 0.22 micron PVDF membrane (Millipore Durapore ®) into a sterile receiving vessel. Fill the drug product into sterile vials. Upon completion of filling, partially stopper the vials and place the vials into the lyophilizer. Upon completion of the lyophilization cycle (about 3-4 days), fully stopper the vials under a slight vacuum (target 866 mbar) and seal. Store all vials at refrigerated conditions (2-8 °C). The unit formula is shown in Table 3.

Table 3

| Unit Formula Active Ingredient | Quantity (mg/mL) |
|---|---|
| (S)-lactate monohydrate[1] salt | 4.34 (Equivalent to 3.35 free base) |
| Other Ingredients[2] | |
| Trehalose | 23.5 |
| Mannitol | 30.0 |
| Polysorbate 80 | 1.25 |
| Water for Injection | q.s. |
| Lactic Acid or Sodium hydroxide solution[3] | - - |

[1] The amount of active pharmaceutical ingredient may be adjusted to take into account the Assay "as is, free base" of the drug substance.
[2] A reasonable variation of +/-10% is allowed for each excipient unless otherwise stated.
[3] Quantity sufficient to adjust pH.

As used herein, the term "kit" refers to a package comprising at least two separate containers, wherein a first container contains 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, and a second container contains 5-(5-(2-(3-aminopropoxy)-6-methoxyphenyl)-1H-pyrazol-3-ylamino)pyrazine-2-carbonitrile, or a pharmaceutically acceptable salt thereof. A "kit" may also include instructions to administer all or a portion of the contents of these first and second containers to a cancer patient, preferably a squamous histology cancer, breast cancer, prostate cancer, endometrial cancer, ovarian cancer, and/or colorectal cancer patient and, more preferably a squamous NSCLC, HNSCC, esophageal cancer, anal cancer, and/or TNBC patient.

As used herein, the terms "treating," "to treat," or "treatment" refers to restraining, slowing, stopping, reducing, or reversing the progression or severity of an existing symptom, disorder, condition, or disease.

As used herein, the term "patient" refers to a mammal, preferably a human.

As used herein, the terms "cancer" and "cancerous" refer to or describe the physiological condition in patients that is typically characterized by unregulated cell growth. Included in this definition are benign and malignant cancers. By "early stage cancer" or "early stage tumor" is meant a cancer that is not invasive or metastatic or is classified as a Stage 0, I, or II cancer. Examples of cancer include, but are not limited to, squamous histology cancers, including squamous NSCLC, HNSCC, esophageal cancer, and anal cancer, breast cancer, including TNBC, prostate cancer, endometrial cancer, ovarian cancer, and colorectal cancer.

A main advantage of the combination treatments of the invention is the ability of producing marked anti-cancer effects in a patient without causing significant toxicities or adverse effects, so that the patient benefits from the combination treatment method overall. The therapeutic agents used in the invention may cause inhibition of metastatic spread without

shrinkage of the primary tumor, or may simply exert a tumoristatic effect. Because the invention relates to the use of a combination of unique anti-tumor agents, novel approaches to determining efficacy of any particular combination therapy of the present invention can be optionally employed, including, for example, measurement of plasma or urinary markers of angiogenesis and measurement of response through radiological imaging.

As used herein, the term "progressive disease" refers to at least a 20% increase in the sum of the diameters of target lesions, taking as reference the smallest (nadir) sum since the treatment started, or the appearance of one or more new lesions. Requires not only 20% increase, but absolute increase of a minimum of 5 mm over sum.

As used herein, the term "primary tumor" or "primary cancer" is meant the original cancer and not a metastatic lesion located in another tissue, organ, or location in the subject's body.

As used herein, the term "effective amount" refers to the amount or dose of 8-[5-(1-hydroxy-1-methylethyl)pyridin-3-yl]-1-[(2S)-2-methoxypropyl]-3-methyl-1 ,3-dihydro-2H-imidazo[4,5-c]quinolin-2-one, or a pharmaceutically acceptable salt thereof, and to the amount or dose of 5-(5-(2-(3-aminopropoxy)-6-methoxyphenyl)-1H-pyrazol-3-ylamino)pyrazine-2-carbonitrile, or a pharmaceutically acceptable salt thereof which, upon single or multiple dose administration to the patient, provides an effective response in the patient under diagnosis or treatment. It is also understood that a combination therapy of the present invention is carried out by administering 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, together with 5-(5-(2-(3-aminopropoxy)-6-methoxyphenyl)-1H-pyrazol-3-ylamino)pyrazine-2-carbonitrile, or a pharmaceutically acceptable salt thereof in any manner which provides effective levels of 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one or a pharmaceutically acceptable salt thereof, and 5-(5-(2-(3-aminopropoxy)-6-methoxyphenyl)-1H-pyrazol-3-ylamino)pyrazine-2-carbonitrile, or a pharmaceutically acceptable salt thereof in the body.

An effective amount can be readily determined by the attending diagnostician, as one skilled in the art, by the use of known techniques and by observing results obtained under analogous circumstances. In determining the effective amount for a patient, a number of factors are considered by the attending diagnostician, including, but not limited to: the species of patient; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, is generally effective over a wide dosage range in the combination of the present invention. For example, dosages per day normally fall within the range of about 100 mg to about 200 mg twice per day on Days 1 to 14 of a 14-day cycle, preferably about 150 mg to about 200 mg twice per day on Days 1 to 14 of a 14-day cycle, and most preferably about 200 mg twice per day on Days 1 to 14 of a 14-day cycle. In addition, 5-(5 -(2-(3 -aminopropoxy)-6-methoxyphenyl)-1H-pyrazol-3 -ylamino)pyrazine-2-carbonitrile, or a pharmaceutically acceptable salt thereof, is generally effective over a wide dosage range in the combination of the present invention. For example, dosages on Day 1 of a 14-day cycle, or every 14 or 21 days, or on Day 1 and Day 15 of a 28-day cycle normally fall within the range of about 60 $mg/m^2$ to about 105 $mg/m^2$, more preferably about 80 $mg/m^2$ to about 105 $mg/m^2$, even more preferably about 90 $mg/m^2$ to about 105 $mg/m^2$, and most preferably about 105 $mg/m^2$. Also, the skilled artisan will appreciate that 5 -(5-(2-(3-aminopropoxy)-6-methoxyphenyl)-1H-pyrazol-3-ylamino)pyrazine-2-carbonitrile, or a pharmaceutically acceptable salt thereof, can be administered at a dose of about 60 $mg/m^2$ to about 105 $mg/m^2$ in discrete dosages within this range, at increments of 60 $mg/m^2$, 61 $mg/m^2$, 62 $mg/m^2$, 63 $mg/m^2$ and so forth in single, 1 $mg/m^2$ increments up to and including 105 $mg/m^2$. Additionally, preferred dosages include 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, administered at a dose of about 100 mg to about 200 mg twice per day on Days 1 to 14 of a 14-day cycle and 5-(5-(2-(3-aminopropoxy)-6-methoxyphenyl)-1H-pyrazol-3-ylamino)pyrazine-2-carbonitrile, or a pharmaceutically acceptable salt thereof, administered at a dose of about 60 $mg/m^2$, 80 $mg/m^2$, 90 $mg/m^2$, or 105 $mg/m^2$ on Day 1 of a 14-day cycle, or every 14 or 21 days, or on Day 1 and Day 15 of a 28-day cycle. More preferably, 8-[5-(1-hydroxy-1-methylethyl)pyridin-3 -yl] -1 - [(2S)-2-methoxypropyl]-3-methyl-1,3-dihydro-2H-imidazo[4,5-c]quinolin-2-one, or a pharmaceutically acceptable salt thereof, is administered at a dose of about 150 mg to about 200 mg twice per day on Days 1 to 14 of a 14-day cycle and 5-(5-(2-(3-aminopropoxy)-6-methoxyphenyl)-1H-pyrazol-3-ylamino)pyrazine-2-carbonitrile, or a pharmaceutically acceptable salt thereof, is administered at a dose of about 80 $mg/m^2$, 90 $mg/m^2$, or 105 $mg/m^2$ on Day 1 of a 14-day cycle, or every 14 or 21 days, or on Day 1 and Day 15 of a 28-day cycle. Even more preferably, 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, is administered at a dose of about 200 mg twice per day on Days 1 to 14 of a 14-day cycle and 5-(5-(2-(3-aminopropoxy)-6-methoxyphenyl)-1H-pyrazol-3 -ylamino)pyrazine-2-carbonitrile, or a pharmaceutically acceptable salt thereof, is administered at a dose of about 105 $mg/m^2$ on Day 1 of a 14-day cycle, or every 14 or 21 days, or on Day 1 and Day 15 of a 28-day cycle. As an alternate preference, 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, is administered at a dose of about 100 mg twice per day on Days 1 to 14 of a 14-day cycle and 5-(5-(2-(3-aminopropoxy)-6-methoxyphenyl)-1H-pyrazol-3-ylamino)pyrazine-2-carbonitrile, or a pharmaceutically acceptable salt thereof, is administered at a dose of about 60 mg/$m^2$ every 21 days or on Day 1 and Day 15 of a 28-day cycle. As a further alternate preference, 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, is administered at a dose of about 100 mg twice per day on Days 1 to 14 of a 14-day cycle and 5-(5-(2-(3-aminopropoxy)-6-methoxyphenyl)-1H-pyrazol-3-ylamino)pyrazine-2-carbonitrile, or a pharmaceutically acceptable salt thereof, is administered at a dose of about 105 mg/$m^2$ every 21 days or on Day 1 and Day 15 of a 28-day cycle.

Dosages as described above are useful when administering 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, and 5-(5-(2-(3-aminopropoxy)-6-methoxyphenyl)-1H-pyrazol-3-ylamino)pyrazine-2-carbonitrile, or a pharmaceutically acceptable salt thereof, in combination. In some instances dosage levels below the lower limit of the aforesaid ranges for 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, and 5-(5-(2-(3-aminopropoxy)-6-methoxyphenyl)-1H-pyrazol1-3-ylamino)pyrazine-2-carbonitrile, or a pharmaceutically acceptable salt thereof, may be more than adequate, while in other cases smaller or still larger doses may be acceptably employed, and therefore the above dosage range is not intended to limit the scope of the invention in any way. Additional cycles can be utilized as needed for treatment of the patient in need thereof.

The free base compound, 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, is preferred. It will be understood by the skilled reader that 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one and 5-(5-(2-(3-aminopropoxy)-6-methoxyphenyl)-1H-pyrazol-3-ylamino)pyrazine-2-carbonitrile are capable of forming salts. A salt form of 5-(5-(2-(3-aminopropoxy)-6-methoxyphenyl)-1H-pyrazol-3-ylamino)pyrazine-2-carbonitrile is preferred, more particularly, the monomesylate monohydrate form or the lactate monohydrate form. 8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one and 5-(5-(2-(3-aminopropoxy)-6-methoxyphenyl)-1H-pyrazol-3-ylamino)pyrazine-2-carbonitrile can react with any of a number of inorganic and organic acids to form pharmaceutically acceptable acid addition salts. Such pharmaceutically acceptable acid addition salts and common methodology for preparing them are well known in the art. See, *e.g.*, P. Stahl, et al., HANDBOOK OF PHARMACEUTICAL SALTS: PROPERTIES, SELECTION AND USE, (VCHA/Wiley-VCH, 2002); L.D. Bighley, S.M. Berge, D.C. Monkhouse, in "Encyclopedia of Pharmaceutical Technology'. Eds. J. Swarbrick and J.C. Boylan, Vol. 13, Marcel Dekker, Inc., New York, Basel, Hong Kong 1995, pp. 453-499; S.M. Berge, et al., "Pharmaceutical Salts", Journal of Pharmaceutical Sciences, Vol 66, No. 1, January 1977.

8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, and 5-(5-(2-(3-aminopropoxy)-6-methoxyphenyl)-1H-pyrazol-3-ylamino)pyrazine-2-carbonitrile, or a pharmaceutically acceptable salt thereof are preferably formulated as pharmaceutical compositions administered by any route which makes the compound bioavailable. The route of administration may be varied in any way, limited by the physical properties of the drugs and the convenience of the patient and the caregiver. Preferably, 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, is administered orally. Alternatively, 8-[5-(1-hydroxy-1-methylethyl)pyridin-3-yl]-1-[(2S)-2-methoxypropyl]-3-methyl-1 ,3-dihydro-2H-imidazo[4,5-c]quinolin-2-one, or a pharmaceutically acceptable salt thereof, is formulated for parenteral administration, such as intravenous or subcutaneous administration. Preferably, 5-(5-(2-(3-aminopropoxy)-6-methoxyphenyl)-1H-pyrazol-3-ylamino)pyrazine-2-carbonitrile, or a pharmaceutically acceptable salt thereof is formulated for parenteral administration, such as intravenous or subcutaneous administration. Most preferably, 5-(5-(2-(3-aminopropoxy)-6-methoxyphenyl)-1H-pyrazol-3-ylamino)pyrazine-2-carbonitrile, or a pharmaceutically acceptable salt thereof is formulated for intravenous administration. Such pharmaceutical compositions and processes for preparing same are well known in the art. (See, e.g., Remington: The Science and Practice of Pharmacy, L.V. Allen, Editor, 22nd Edition, Pharmaceutical Press, 2012).

As used herein, the phrase "in combination with" refers to the administration of 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, with 5-(5-(2-(3-aminopropoxy)-6-methoxyphenyl)-1H-pyrazol-3-ylamino)pyrazine-2-carbonitrile, or a pharmaceutically acceptable salt thereof simultaneously. As used herein, the phrase "in combination with" also refers to the administration of 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, with 5-(5-(2-(3-aminopropoxy)-6-methoxyphenyl)-1H-pyrazol-3-ylamino)pyrazine-2-carbonitrile, or a pharmaceutically acceptable salt thereof sequentially in any order. As used herein, the phrase "in combination with" also refers to the administration of 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, or a pharmaceutically acceptable salt thereof, with 5-(5-(2-(3-aminopropoxy)-6-methoxyphenyl)-1H-pyrazol-3-ylamino)pyrazine-2-carbonitrile, or a pharmaceutically acceptable salt thereof in any combination thereof. 8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one can be administered prior to administration of 5-(5-(2-(3-aminopropoxy)-6-methoxyphenyl)-1H-pyrazol-3-ylamino)pyrazine-2-carbonitrile, or a pharmaceutically acceptable salt thereof. 8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one can be administered at the same time as administration of 5-(5-(2-(3-aminopropoxy)-6-methoxyphenyl)-1H-pyrazol-3-ylamino)pyrazine-2-carbonitrile, or a pharmaceutically acceptable salt thereof. 8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one can be administered subsequent to administration of 5-(5-(2-(3-aminopropoxy)-6-methoxyphenyl)-1H-pyrazol-3-ylamino)pyrazine-2-carbonitrile, or a pharmaceutically acceptable salt thereof. 8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one can be administered prior to, at the same time as, or subsequent to administration of 5-(5-(2-(3-aminopropoxy)-6-methoxyphenyl)-1H-pyrazol-3-ylamino)pyrazine-2-carbonitrile, or a pharmaceutically acceptable salt thereof, or in some combination thereof.

Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered prior to each administration of 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered at the same time as each administration of 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered subsequent to each administration of 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered prior to, at the same time as, or subsequent to, each administration of 5-(5-(2-(3-aminopropoxy)-6-methoxyphenyl)-1H-pyrazol-3-ylamino)pyrazine-2-carbonitrile, or a pharmaceutically acceptable salt thereof or some combination thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered at different intervals in relation to therapy with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered in a single or series of dose(s) prior to, at any time during, or subsequent to the course of treatment with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol1-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1 -[(2S)-2-methoxypropyl]-3-methyl-1,3-dihydro-2H-imidazo[4,5-c]quinolin-2-one can be administered in a single dose prior to, at any time during, or subsequent to the course of treatment with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered in a single dose prior to the course of treatment with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered in a single dose at any time during the course of treatment with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered in a single dose subsequent to the course of treatment with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered in a series of doses prior to the course of treatment with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered in a series of doses subsequent to the course of treatment with 5-(5-(2-(3-aminopropoxy)-6-methoxyphenyl)-1H-pyrazol-3-ylamino)pyrazine-2-carbonitrile, or a pharmaceutically acceptable salt thereof. Where 5-(5-(2-(3-aminopropoxy)-6-methoxyphenyl)-1H-pyrazol-3-ylamino)pyrazine-2-carbonitrile, or a pharmaceutically acceptable salt thereof is administered at repeated intervals (e.g. during a standard course of treatment), 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be administered in a series of doses subsequent to the course of treatment with 5-(5-(2-(3-aminopropoxy)-6-methoxyphenyl)-1H-pyrazol-3-ylamino)pyrazine-2-carbonitrile, or a pharmaceutically acceptable salt thereof.

The compound 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one can be made, for example, according to the disclosure in WO 2012/097039. The compound 5-(5-(2-(3-aminopropoxy)-6-methoxyphenyl)-1H-pyrazol-3 -ylamino)pyrazine-2-carbonitrile can be made, for example, according to the disclosure in WO 2010/077758. The compound 5-(5-(2-(3-aminopropoxy)-6-methoxyphenyl)-1H-pyrazol-3-ylamino)pyrazine-2-carbonitrile can be used as the methanesulfonate hydrate form, i.e. 5-(5-(2-(3-aminopropoxy)-6-methoxyphenyl)-1H-pyrazol-3-ylamino)pyrazine-2-carbonitrile methanesulfonate hydrate. Alternative compound names for this form include 2-pyrazinecarbonitrile, 5-[[5-[2-(3-aminopropyl)-6-methoxyphenyl]-1H-pyrazol-3-yl]amino] monomesylate monohydrate. Additionally, the compound 5-(5-(2-(3-aminopropoxy)-6-methoxyphenyl)-1H-pyrazol-3 -ylamino)pyrazine-2-carbonitrile can be used as the lactate monohydrate form, i.e. 5-(5-(2-(3-aminopropoxy)-6-methoxyphenyl)-1H-pyrazol-3-ylamino)pyrazine-2-carbonitrile (S)-lactate monohydrate, as detailed herein above..

As used herein, the following terms have the meanings indicated: "AUC" refers to area under the curve; "AE" refers to adverse effect; "BID" refers to twice a day dosing; "DLT" refers to dose limiting toxicity; "EAR" refers to Expected Additive Response; "HEC" refers to hydroxyethyl cellulose; "IV" refers to intravenous; "MTD" refers to maximum tolerated dose; "PDX" refers to patient-derived xenograft; "PO" refers to oral dosing (per os); "q.s." refers to quantum sufficit; and "SC" refers to subcutaneous dosing.

Antitumor Effects of 5-(5-(2-(3-Aminopropoxy)-6-methoxyphenyl)-1H-pyrazol-3-ylamino)pyrazine-2-carbonitrile methanesulfonate hydrate in Combination with 8-[5-(1-Hydroxy-1-methylethyl)pyridin-3-yl]-1-[(2S)-2-methoxypropyl]-3-methyl-1,3-dihydro-2H-imidazo[4,5-c]quinolin-2-one in PDX Mouse Models of TNBC

Tumor Implantation and Treatment

Establish PDX tumor models from viable human tumor tissue and serially passage in immunocompromised female mice a limited number of times. Using tumor fragments harvested from donor host animals, perform unilateral subcutaneous implants from a specific passage of the PDX tumor model on the flanks of the experimental animals. When tumor volumes reach approximately 150-250 $mm^3$ in size, randomize the animals by treatment arm by randomization techniques well known in the art and place into their respective treatment groups using 1 animal per treatment group (3 animals in an untreated group). Formulate CHK1 inhibitor, 5-(5-(2-(3-aminopropoxy)-6-methoxyphenyl)-1H-pyrazol-3-ylamino)pyrazine-2-carbonitrile methanesulfonate hydrate, (Compound A) every 14 days in 20% captisol (dissolved in water) and administer subcutaneously twice daily at a dose of 10 mg/kg on days 1/2/3 of a weekly cycle for 4 weeks (SC, BID [3 days on/4 off] $\times$4). Formulate the PI3K/mTOR dual inhibitor, 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one, (Compound B) weekly in 1% (HEC) / 0.25% Tween 80 (Polysorbate 80) / 0.05% Dow-Corning Antifoam 1510-US and administer by oral gavage twice-daily for 28 days (PO, BID $\times$ 28) at a dose of 7.5 mg/kg. Dose combination treatment animals with both compounds according to the schedule described above for monotherapy, and dose vehicle treatment animals with vehicle according to the schedule for Compound B.

Test the combination of Compound A and Compound B, as well as their respective monotherapies, in 40 PDX models (Table 1) of TNBC. Assess antitumor efficacy of the treatment groups by measuring tumor volume via caliper measurements twice a week during the course of the study. Measure body weight twice weekly during the course of the study as a general indicator of tolerability.

Data Capture and Analysis

Capture tumor size and body weight twice per week. Estimate tumor volume (V) by using the formula: V = (π/6) x L x $W^2$ where L = larger of measured diameter and W = smaller perpendicular diameter. Calculate relative tumor volume using the tumor volume measurement taken nearest to the last day of dosing for each animal (*T*), divided by the baseline tumor volume for that animal ($T_0$), which is the volume recorded on or just prior to the first day of dosing, $Y = T/T_0$. Calculate %ΔT/ΔC values using the formula $\%\Delta T/\Delta C = 100(Y_T - 1)/(Y_C - 1)$, whereby $Y_T$ or $Y_C$ = geometric mean relative volume of the compound treated group or the control group, respectively. Tumor growth inhibition is observed in those instances where the calculated values for %ΔT/ΔC are less than 100% whereby greater inhibition results in smaller %ΔT/ΔC values. If ΔT <0, then a tumor regression value is calculated instead of %ΔT/ΔC whereby % Regression = $100(Y_T - 1)$. Note that negative values indicate regression. We collectively refer to %ΔT/ΔC and % Regression as % Response.

Examine relative tumor volume (final volume divided by baseline volume) on the last day of treatment or the last day on which data from all three treatment groups and vehicle is available, if it is earlier than the last day of treatment and at least 14 days after baseline. Expected additive relative volume is determined using the Bliss method (Bliss, C. I., Ann Appl Biol, 1939; 26(3):585-615). Specifically, denoting Y = relative volume, $Y_{exp} = (Y_1 * Y_2)/Y_V$, where $Y_{Exp}$ = expected additive relative volume, $Y_1$ and $Y_2$ are the relative volumes in the single agent groups (Compound A and Compound B), and $Y_V$ is the relative volume in the vehicle group. A range of additivity is defined around the expected additive relative volume via upper and lower limits as follows:

$$Y_{upper} = \max(2 * Y_{Exp}, Y_{Exp} + 0.2)$$

$$Y_{lower} = \min(Y_{Exp}/2\ ,\ \ Y_{Exp} - 0.2)$$

The upper limit of the range is the larger of 2-fold above or 20% above the expected additive relative volume, and the lower limit is the smaller of 2-fold below or 20% below the expected additive relative volume.

If the observed combination relative volume is within this range, the combination is declared additive. If it is below the lower limit, the combination is declared synergistic. If it is above the upper limit, and the combination relative volume is lower than at least one of the single agent relative volumes, the combination is declared less than additive; otherwise, it is declared antagonistic.

Results:

Compound A monotherapy shows efficacy values ranging from 126.1% to -100% response (Table 4, Column 2, see Figure 1). Compound B monotherapy shows efficacy values ranging from 163% to -31% response (Table 4, Column 3, see Figure 2). Compound A plus Compound B combination shows efficacy values ranging from 121.2% to -100% response (Table 4, Column 4, see Figure 3). The combination group demonstrates actual % response values lower than either monotherapy (greater efficacy) in 25 of 38 evaluable models and additivity or better in 31 of 38 evaluable models, while in 9 of the 25 models the combination shows synergy as defined above.

Table 4: Monotherapy and Combination Therapy Efficacy (versus Vehicle) of Compounds A and B.

| PDX TNBC Model | A % Rsp. | B % Rsp. | Combo % Rsp. | EAR % Rsp. | EAR % Rsp. Lower | EAR % Rsp. Upper | Combo Effect Category |
|---|---|---|---|---|---|---|---|
| CTG-0012 | 33.4 | 49.0 | 6.0 | 11.2 | -18.8 | 40.3 | Additive |
| CTG-0017 | 52.3 | 28.1 | 65.6 | 9.3 | -25.0 | 37.2 | Antagonistic |
| CTG-0018 | -57.6 | 86.0 | -47.7 | -61.5 | -81.5 | -22.9 | Additive |
| CTG-0052 | 41.3 | 8.6 | 28.0 | -23.8 | -61.9 | 8.2 | < Additive |
| CTG-0437 | -87.5 | 80.3 | -100.0 | -89.6 | -100.0 | -69.6 | Additive |
| CTG-0670 | 112.8 | 117.6 | -8.0 | 100.0 | 13.8 | 272.5 | Synergy |
| CTG-0869 | 52.6 | 64.9 | 28.4 | 32.7 | 12.0 | 74.3 | Additive |
| CTG-0986 | 32.2 | 54.6 | 23.4 | 14.1 | 0.7 | 40.9 | Additive |

(continued)

| PDX TNBC Model | A % Rsp. | B % Rsp. | Combo % Rsp. | EAR % Rsp. | EAR % Rsp. Lower | EAR % Rsp. Upper | Combo Effect Category |
|---|---|---|---|---|---|---|---|
| CTG-1019 | 86.6 | 158.0 | 121.2 | 100.0 | 39.1 | 221.7 | Additive |
| CTG-1106 | -39.0 | -31.0 | -49.0 | -86.2 | -100.0 | -66.2 | < Additive |
| CTG-1151 | -84.6 | 92.5 | -70.7 | -85.3 | -100.0 | -65.3 | Additive |
| CTG-1153 | -23.6 | 86.5 | -73.4 | -32.3 | -66.2 | 6.4 | Synergy |
| CTG-1167 | 62.3 | 65.8 | -36.0 | 39.1 | 11.4 | 94.7 | Synergy |
| CTG-1242 | 66.8 | 107.3 | 25.6 | 71.9 | 31.2 | 153.2 | Synergy |
| CTG-1340 | 41.0 | 17.1 | 17.0 | -12.1 | -56.1 | 20.1 | Additive |
| CTG-1350 | 20.3 | 7.7 | -27.8 | -47.7 | -73.8 | 2.0 | Additive |
| CTG-1408 | 11.1 | 27.5 | -8.0 | -38.1 | -69.1 | 9.9 | Additive |
| CTG-1453 | 2.3 | -6.9 | 1.5 | -60.4 | -80.4 | -20.8 | < Additive |
| CTG-1520 | 49.9 | 61.2 | 27.2 | 29.5 | 12.0 | 64.5 | Additive |
| MAXF 1384 | 52.8 | 60.3 | -84.9 | 29.1 | 6.0 | 75.3 | Synergy |
| MAXF 449 | -100.0 | 34.7 | -45.5 | -100.0 | -100.0 | -77.2 | < Additive |
| MAXF 508 | -100.0 | 41.3 | -81.1 | -100.0 | -100.0 | -77.7 | Additive |
| MAXF 574 | 22.9 | 59.4 | -75.8 | 9.3 | -21.1 | 34.6 | Synergy |
| MAXF 583 | 9.6 | 121.1 | -55.0 | 19.1 | -35.1 | 102.3 | Synergy |
| MAXF 857 | 32.4 | 72.7 | -0.3 | 16.2 | -37.9 | 99.0 | Additive |
| MAXF MX1 | 126.1 | 163.0 | 64.4 | 100.0 | 42.5 | 215.1 | Additive |
| ST035 | 2.1 | 54.0 | 0.0 | -25.5 | -62.8 | 3.4 | Additive |
| ST069 | -79.6 | 112.0 | -100.0 | -77.6 | -100.0 | -55.1 | Additive |
| ST1077 | 0.0 | 9.1 | -83.7 | -80.2 | -100.0 | -60.2 | Additive |
| ST1283 | 0.0 | 23.3 | 0.0 | -68.4 | -88.4 | -36.7 | < Additive |
| ST2057 | 44.8 | 26.9 | 11.2 | 6.0 | -33.1 | 29.7 | Additive |
| ST231 | -74.6 | 76.8 | -81.3 | -79.6 | -100.0 | -59.2 | Additive |
| ST319 | 8.9 | 40.3 | -83.7 | -27.9 | -63.9 | 9.3 | Synergy |
| ST430 | 80.9 | 59.2 | 60.6 | 47.1 | 17.8 | 105.7 | Additive |
| ST518 | -23.4 | 66.5 | -74.0 | -44.1 | -72.0 | 2.9 | Synergy |
| ST575 | -87.5 | 51.3 | -81.3 | -100.0 | -100.0 | -72.4 | Additive |
| ST821 | -81.9 | 78.5 | -81.3 | -85.0 | -100.0 | -65.0 | Additive |
| ST996 | 0.7 | 23.5 | 15.6 | -68.1 | -88.1 | -36.2 | < Additive |
| CTG-0888 | 15.0 | 21.2 | ND | -28.0 | -64.0 | 4.2 | ND |
| ST848 | -50.0 | 24.6 | ND | -73.9 | -93.9 | -47.7 | ND |

Abbreviations: %Rsp = %$\Delta T/\Delta C$ or %Regression; Combo = Combination; EAR = Expected Additive Response; ND = Not Determined due to early body weight loss.

A Phase 1b Trial of 5-[[5-[2-(3-aminopropyl)-6-methoxyphenyl]-1H-pyrazol-3-yl]amino] monomesylate monohydrate (Compound A in this section) in combination with 8-[5-(1-hydroxy-1-methylethyl)pyridin-3-yl]-1-[(2S)-2-methoxypropyl]-3-methyl-1,3-dihydro-2H-imidazo[4,5-c]quinolin-2-one (Compound B in this section) in Advanced and/or Metastatic Tumors

Study Design

The study (I4D-MC-JTJF [JTJF]) is a Phase 1b multicenter, nonrandomized, open-label, dose escalation clinical study of the combination of Compound A and Compound B in patients with advanced and/or metastatic cancer.

- Escalation of Compound A and Compound B: define the MTD of Compound A and Compound B when administered in combination in patients with advanced and/or metastatic cancer when Compound B is administered BID on Days 1 to 14 and Compound A is administered on Day 1 of a 14-day cycle.
- Compound A and Compound B expansion: dose expansion cohort to further assess the safety profile of Compound A when administered in combination with Compound B in patients with advanced and/or metastatic cancer when Compound B is administered BID on Days 1 to 14 and Compound A is administered on Day 1 of a 14-day cycle.

The dose escalations will be driven by safety using a modified 3+3 scheme, with incorporation of a Bayesian model-based dose escalation method (Neuenschwander et al., Statist. Medicine (2008) 27, 2420-2439) to assist in estimation of dose limiting toxicity rate at recommended dose levels. As the maximum tolerated dose is identified, an expansion cohort will be initiated.

Study Objectives

The primary objective of this study is to determine a recommended Phase 2 dose of Compound A and Compound B in combination that can be safely administered to patients with advanced and/or metastatic cancer. The secondary objectives of this study are 1) to characterize the safety and toxicity profile of Compound A and Compound B in combination, 2) to characterize the pharmacokinetics of Compound A and Compound B in combination, and 3) to document any antitumor activity observed in patients with advanced and/or metastatic cancer during dose escalation and expansion with Compound A in combination with Compound B. The exploratory objectives of this study are 1) to identify exploratory biomarkers associated with response and safety, and 2) to study change in tumor size in association with other efficacy outcomes in patients receiving Compound A in combination with Compound B.

Treatment Plan

The initial cohort of patients will be dosed with Compound A (60 mg/$m^2$ administered by IV infusion on Day 1 of a 14-day cycle) and Compound B (100 mg orally BID on Days 1 to 14 of a 14-day cycle). The IV infusion will occur over approximately 60 to 70 minutes. A $\pm$10% variance between the calculated total dose of Compound A and the dose administered is allowed for ease of dose administration.

The administration of Compound B will follow the end of Compound A infusion (+5 minutes) on Day 1 of each cycle. Patients should take the morning and evening doses of Compound B approximately 12 hours apart (preferably within a 10- to 14-hour range). Clinic personnel will instruct patients to take Compound B at approximately the same time each dosing day with a full glass of water. Patients should not consume food for approximately 1 hour before taking each dose of Compound B. Patients should arrive at the clinic in a fasted state on Day 1 of each cycle so the predose assessments (such as laboratory tests) are performed with the patient in a fasted state.

If neutropenia or neutropenia-related events are identified as a DLT for the combination of Compound A and Compound B, required prophylactic administration of G-CSF may be instituted following Compound A administration, even in Cycle 1. This decision will be made following discussions with the investigators and the CRP/CRS and will be documented in writing.

The doses of both compounds will be escalated until the MTD of the combination is defined. Following the dose escalation, an expansion cohort will assess Compound A in combination with Compound B in patients with advanced or metastatic cancer at the recommended Phase 2 dose.

Dose Escalation

Dose escalations will be driven by a modified 3+3 paradigm, using a model-based dose escalation method (Neuenschwander et al., Statist. Medicine (2008)) to provide quantitative guidance on the determination of the dose

level for the next enrolled patients. This method incorporates the prior expectations of the dose-toxicity curve and the observed DLT data from the ongoing study into the dose level determination, with control of over-dosing probability.

Prior expectations of the dose-toxicity curve for combinations of both agents (based on monotherapy profiles) will be incorporated into the model to provide a recommendation for the respective doses of each agent at the next dose level. Based on the model prediction, one or both agents may be escalated, but the escalation will not exceed the monotherapy MTD for each agent.

A cohort will initially plan to include 3 patients at a dose level. If a patient experiences a DLT, up to 3 additional patients will be enrolled at the same dose level. The model-based dose escalation method will be applied to the observed data on an ongoing basis throughout the dose escalation. A decision to escalate or de-escalate can be made at any time after 3 or more patients have been treated at the current dose level and evaluated for DLTs. If more than 1 patient experiences a DLT at the current dose level, dose escalation will cease and either the MTD will be declared as the previous dose level or additional patients may be treated at intermediate doses between the previous dose level and the current dose level.

Dose Expansion

The expansion cohort will include approximately 9 to 12 evaluable patients with advanced and/or metastatic cancer. The dose in the expansion cohort will not exceed the MTD of either compound as defined during dose escalation. The exact sample size of the expansion cohort will be dependent on the number of patients treated at the combination MTD during escalation and will be adjusted so that a total of approximately 15 patients in the escalation and expansion will be treated at the MTD.

If DLT-equivalent toxicity occurs in one-third or more of patients during Cycle 1 (with a minimum of 6 patients enrolled in the expansion cohort), the enrollment of new patients will cease until the severity and nature of the toxicity is assessed. A data review will be performed to determine whether to continue at the current dose or whether the dose of either compound should be reduced.

At the end of this expansion, a recommended Phase 2 dose of Compound A and Compound B in combination will be agreed upon.

Dose Adjustments and Delays

If a nonhematologic toxicity requires a dose reduction of Compound B, the investigator may also dose reduce Compound A to the next dose level. If a dose reduction of Compound B already occurred and the Compound A dose was not reduced, and another instance of the same toxicity requiring a Compound B dose reduction is observed, then both Compound A and Compound B should be dose reduced.

Dosing delays due to Compound B treatment emergent AEs are permissible up to 2 weeks to allow sufficient time for recovery. Compound B dosing delay beyond 2 weeks may be permissible, if AEs are not considered to be primarily related to Compound B treatment and the investigator deems continuation to have clinical benefit for the patient. Re-escalation to the previous dose is acceptable in the absence of continuing toxicity, provided that this dose is not greater than current dose being tested. If subsequent dose reduction is required after re-escalation, the patient must be maintained at the reduced dose level for all remaining cycles.

Treatment Compliance

Compound A will be administered intravenously at the investigational site, under the direction of the investigator. As a result, a patient's compliance with study drug administration is ensured. Patients should attend scheduled clinic visits and must comply with study criteria under their control.

Patient compliance with Compound B will be assessed at each visit by direct questioning, counting returned capsules and reviewing the patient diary.

The patient must take ≥80% of the intended doses in each cycle to be deemed compliant with study drug administration. Similarly, a patient may be considered noncompliant if he or she is judged by the investigator to have intentionally or repeatedly taken more than the prescribed amount of drug.

Safety Analyses

All patients who receive at least 1 dose of Compound A or Compound B will be evaluated for safety and toxicity. Adverse event terms and severity grades will be assigned by the investigator using CTCAE, Version 4.0.
Safety analyses will include summaries of the following:

- adverse events, including severity and possible relationship to study drug
- dose adjustments
- laboratory values
- vital signs
- DLTs at each dose level and DLT-equivalent toxicities
- ECG readings

Pharmacokinetic Analyses

Pharmacokinetic (PK) analyses will be conducted on patients who receive at least 1 dose of Compound A and Compound B and have had samples collected for PK analysis. PK parameter estimates (for example, AUC, $C_{max}$, CL, CL/F, $CL_R$, $V_{ss}$, $V_{ss}$/F, $t_{1/2}$, etc.) for Compound A and Compound B that can be calculated from the data will be calculated by standard noncompartmental methods of analysis. The noncompartmental Compound A PK parameter estimates in combination with Compound B during Cycles 1 and 2 will be compared to historical Compound A data to assess the potential influence of Compound B administration on the PK of Compound A. In addition, the PK parameter estimates of Compound B will be compared to historical data to determine the potential influence of Compound A on the PK of Compound B.

The Compound A PK parameter estimates will also be used to evaluate dose proportionality provided sufficient data exist from the dose escalation portions of the study. The degree of dose proportionality for Compound A will be assessed by fitting a power model to both AUC and $C_{max}$ (that is, end of infusion) versus dose after single administration during the dose-escalation portions from Cycle 1. Dose proportionality will be assumed for any dose ratio in which the 90% confidence limits of the ratio of dose-normalized geometric means for the same ratio of doses fall within the criteria (0.8, 1.25).

The total Compound B PK parameters estimates will be used to assess the potential effect of Compound A on the PK of each agent by comparison to the known population PK parameters for each agent.

Interim review of the clinical PK data during and the end of dose escalation will be used to help guide the Compound A dose escalation and inform dose selection for dose expansions. It will also be used to begin development of a human population-based PK model of Compound A in combination with Compound B that will be used to generate post-hoc population based PK parameter estimates for each patient, characterize the observed intra- and interpatient PK variability, and subsequently identify the patient-specific covariates that contribute to the observed PK variability of Compound A when used in combination with Compound B.

Additional exploratory analyses, such as a population-based analyses that examine the relationship between Compound A and Compound B exposure and the relationship between ECG changes (for instance, QTc F, QRS, and PR) and/or exploratory biomarkers associated with response (efficacy) and safety may be performed using validated PK software programs if the data warrant.

Efficacy Evaluations

The study is not designed to make an efficacy assessment. However, any tumor response data will be tabulated. Each patient will be assessed by one or more of the following radiologic tests for tumor measurement:

- Computed tomography (CT) scan
- Magnetic resonance imaging (MRI)
- Chest x-ray
- Positron emission tomography (PET) scan

In rare circumstances, historical radiologic exams for RECIST (Response Evaluation Criteria In Solid Tumors) criteria that are obtained greater than 28 days prior to the first dose may be used, but this decision must be discussed with the sponsor and documented in writing.
Each patient's full extent of disease will also be assessed with:

- Tumor measurement by RECIST 1.1 (Eisenhauer et al., EJC (2009) 45, 228-247)
- Evaluation of tumor markers, if indicated
- Evaluation of performance status (refer to the Eastern Cooperative Group Scale (ECOG))

To confirm objective responses, all lesions should be radiologically assessed, and the same radiologic method used for the initial response determination should be repeated at least 4 weeks following the initial observation of an objective response, using the sample method that was used at baseline. If a patient is discontinued from the study,

repeat radiology assessments may be omitted if clear clinical signs of progressive disease are present.

## Claims

**1.** A compound of the formula:

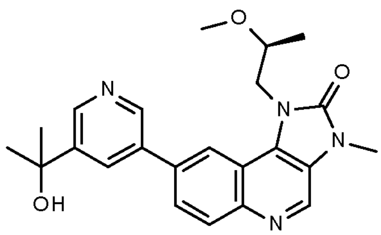

or a pharmaceutically acceptable salt thereof, for simultaneous, separate, or sequential use in combination with a compound of the formula:

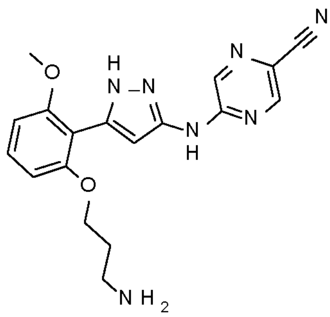

,

or a pharmaceutically acceptable salt thereof, in the treatment of squamous histology cancers, breast cancer, prostate cancer, bladder cancer, cervical cancer, endometrial cancer, ovarian cancer, and colorectal cancer.

**2.** The compound for use of claim 1, wherein the squamous histology cancers and breast cancer are squamous NSCLC, HNSCC, esophageal cancer, anal cancer, and TNBC.

**3.** The compound for use of claim 2, wherein the breast cancer is TNBC.

**4.** The compound for use of claim 1 wherein

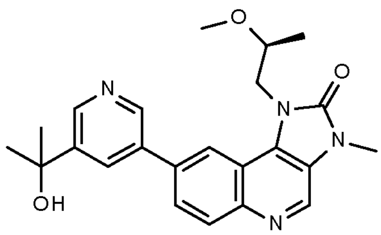

,

or a pharmaceutically acceptable salt thereof, is administered at a dose of about 100 mg twice per day to about 200 mg twice per day on Days 1 to 14 of a 14-day cycle and

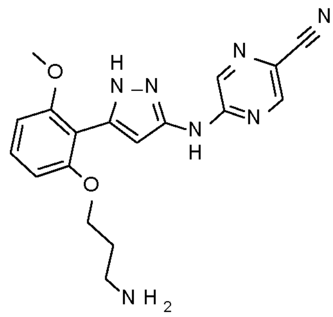

,

or a pharmaceutically acceptable salt thereof, is administered at a dose of about 60 mg/$m^2$ to about 105 mg/$m^2$ on Day 1 of a 14-day cycle or every 14 or 21 days.

**5.** The compound for use of claim 4 wherein

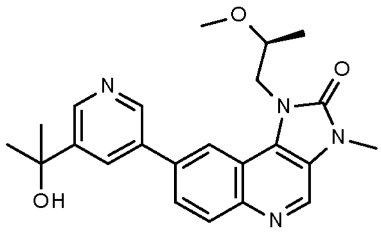

,

or a pharmaceutically acceptable salt thereof, is administered at a dose of about 100 mg twice per day on Days 1 to 14 of a 14-day cycle and

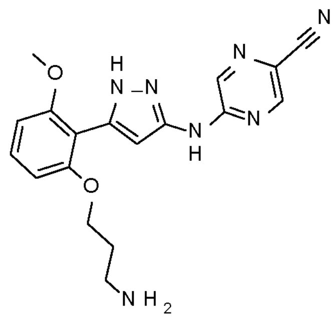

,

or a pharmaceutically acceptable salt thereof, is administered at a dose of about 60 mg/$m^2$ on Day 1 of a 14-day cycle or every 14 or 21 days.

**6.** The compound for use of claim 4 wherein

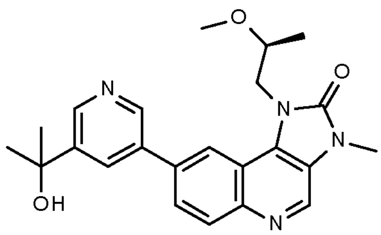

,

or a pharmaceutically acceptable salt thereof, is administered at a dose of about 100 mg twice per day on Days 1 to 14 of a 14-day cycle and

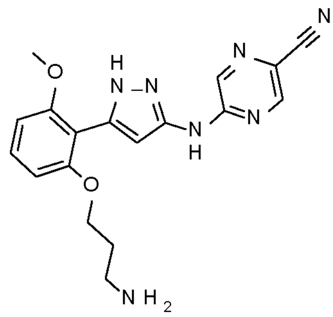

,

or a pharmaceutically acceptable salt thereof, is administered at a dose of about 105 $mg/m^2$ on Day 1 of a 14-day cycle or every 14 or 21 days.

**7.** The compound for use of claim 4 wherein

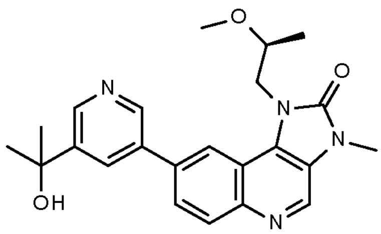

,

or a pharmaceutically acceptable salt thereof, is administered at a dose of about 200 mg twice per day on Days 1 to 14 of a 14-day cycle and

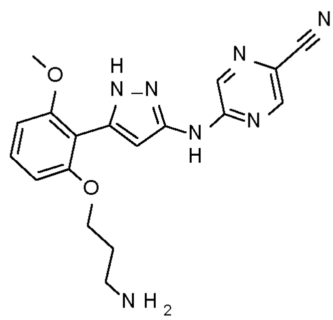

,

or a pharmaceutically acceptable salt thereof, is administered at a dose of about 105 $mg/m^2$ on Day 1 of a 14-day cycle or every 14 or 21 days.

**8.** The compound for use of claims 4-7 wherein

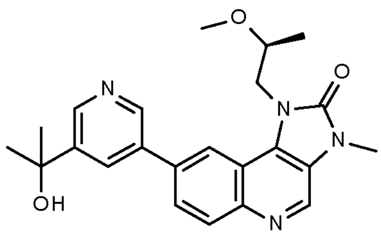

,

or a pharmaceutically acceptable salt thereof, is administered orally and

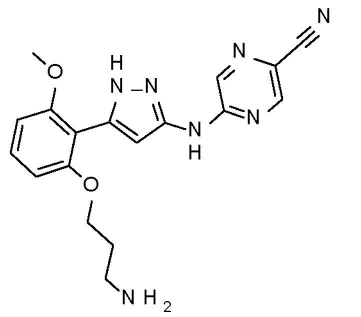

,

or a pharmaceutically acceptable salt thereof, is administered by intravenous infusion.

9. A kit comprising a compound of the formula:

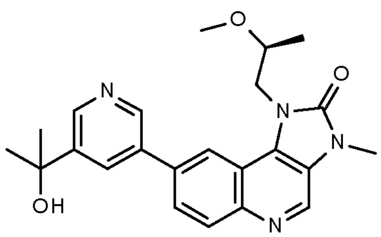

,

or a pharmaceutically acceptable salt thereof, and a compound of the formula:

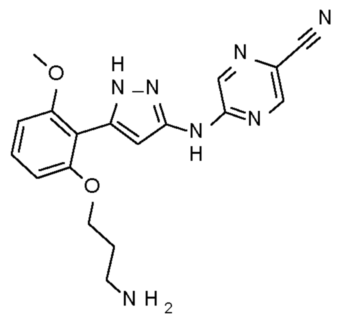

,

or a pharmaceutically acceptable salt thereof, for simultaneous, separate, or sequential use in the treatment of squamous histology cancers, breast cancer, prostate cancer, bladder cancer, cervical cancer, endometrial cancer, ovarian cancer, and colorectal cancer.

10. The kit for use of claim 9, wherein the squamous histology cancers and breast cancer are squamous NSCLC, HNSCC, esophageal cancer, anal cancer, and TNBC.

11. The kit for use of claim 10, wherein the breast cancer is TNBC.

12. A kit, comprising a pharmaceutical composition, comprising a compound of the formula:

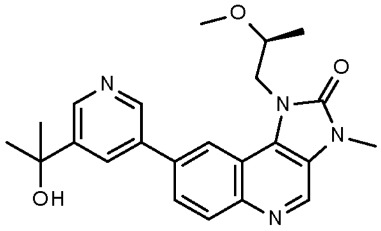

,

or a pharmaceutically acceptable salt thereof, with one or more pharmaceutically acceptable carriers, diluents, or excipients, and a pharmaceutical composition, comprising a compound of the formula:

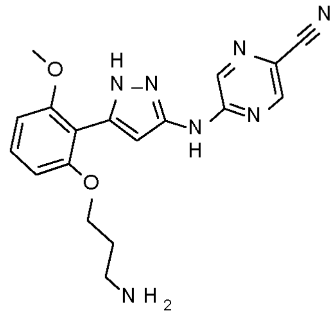

,

or a pharmaceutically acceptable salt thereof, with one or more pharmaceutically acceptable carriers, diluents, or excipients for simultaneous, separate, or sequential use in the treatment of squamous histology cancers, breast cancer, prostate cancer, endometrial cancer, ovarian cancer, and colorectal cancer.

**13.** The kit for use of claim 12, wherein the squamous histology cancers and breast cancer are squamous NSCLC, HNSCC, esophageal cancer, anal cancer, and TNBC.

**14.** The kit for use of claim 13, wherein the breast cancer is TNBC.

**Patentansprüche**

**1.** Verbindung der Formel:

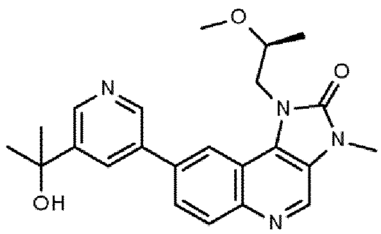

oder ein pharmazeutisch annehmbares Salz davon zur gleichzeitigen, getrennten oder aufeinanderfolgenden Verwendung in Kombination mit einer Verbindung der Formel:

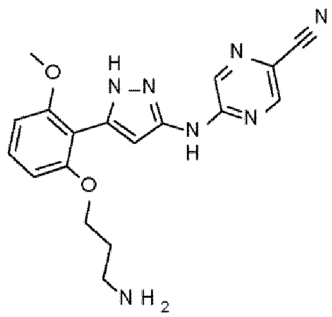

oder einem pharmazeutisch annehmbarem Salz davon bei der Behandlung von Krebsarten mit Plattenepithel-Histologie, Brustkrebs, Prostatakrebs, Blasenkrebs, Gebärmutterhalskrebs, Endometriumkrebs, Eierstockkrebs und kolorektalem Karzinom.

**2.** Verbindung zur Verwendung nach Anspruch 1, wobei die Krebsarten mit Plattenepithel-Histologie und der Brustkrebs Plattenepithel-NSCLC, HNSCC, Speiseröhrenkrebs, Analkrebs und TNBC sind.

**3.** Verbindung zur Verwendung nach Anspruch 2, wobei der Brustkrebs TNBC ist.

**4.** Verbindung zur Verwendung nach Anspruch 1, wobei

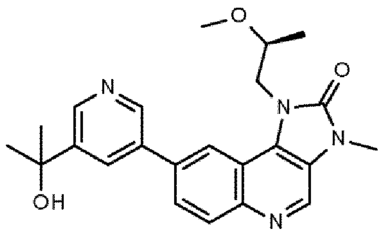

oder ein pharmazeutisch annehmbares Salz davon in einer Dosis von ungefähr 100 mg zweimal pro Tag bis ungefähr 200 mg zweimal pro Tag an den Tagen 1 bis 14 eines 14-tägigen Zyklus verabreicht wird und

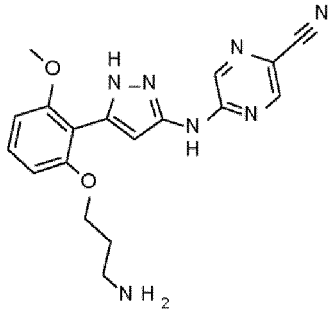

oder ein pharmazeutisch annehmbares Salz davon in einer Dosis von ungefähr 60 $mg/m^2$ bis ungefähr 105 $mg/m^2$ an Tag 1 eines 14-tägigen Zyklus oder alle 14 oder 21 Tage verabreicht wird.

**5.** Verbindung zur Verwendung nach Anspruch 4, wobei

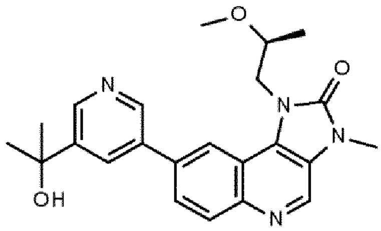

oder ein pharmazeutisch annehmbares Salz davon in einer Dosis von ungefähr 100 mg zweimal pro Tag an den Tagen 1 bis 14 eines 14-tägigen Zyklus verabreicht wird und

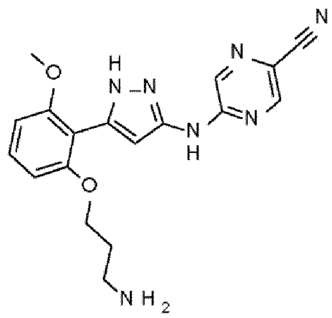

oder ein pharmazeutisch annehmbares Salz davon in einer Dosis von ungefähr 60 $mg/m^2$ an Tag 1 eines 14-tägigen Zyklus oder alle 14 oder 21 Tage verabreicht wird.

**6.** Verbindung zur Verwendung nach Anspruch 4, wobei

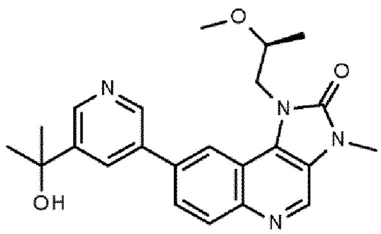

oder ein pharmazeutisch annehmbares Salz davon in einer Dosis von ungefähr 100 mg zweimal pro Tag an den Tagen 1 bis 14 eines 14-tägigen Zyklus verabreicht wird und

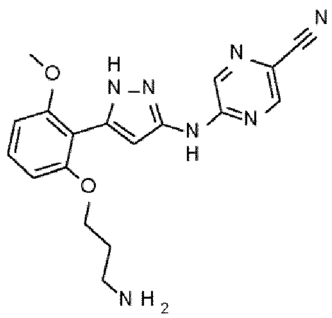

oder ein pharmazeutisch annehmbares Salz davon in einer Dosis von ungefähr 105 $mg/m^2$ an Tag 1 eines 14-tägigen Zyklus oder alle 14 oder 21 Tage verabreicht wird.

**7.** Verbindung zur Verwendung nach Anspruch 4, wobei

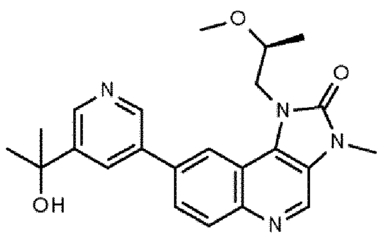

oder ein pharmazeutisch annehmbares Salz davon in einer Dosis von ungefähr 200 mg zweimal pro Tag an den Tagen 1 bis 14 eines 14-tägigen Zyklus verabreicht wird und

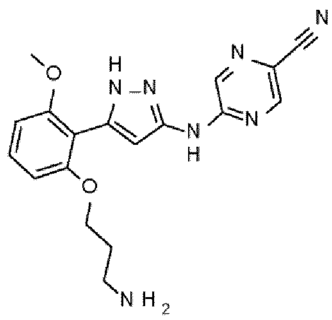

oder ein pharmazeutisch annehmbares Salz davon in einer Dosis von ungefähr 105 $mg/m^2$ an Tag 1 eines 14-tägigen Zyklus oder alle 14 oder 21 Tage verabreicht wird.

**8.** Verbindung zur Verwendung nach Anspruch 4-7, wobei

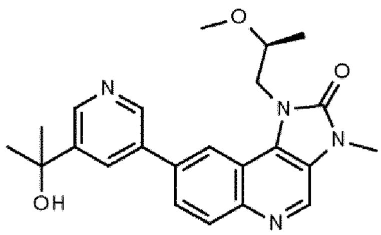

oder ein pharmazeutisch annehmbares Salz davon oral verabreicht wird und

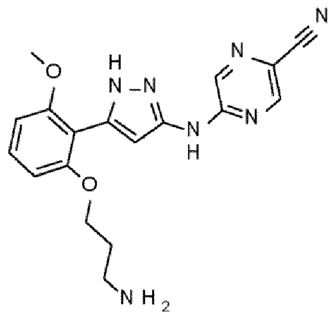

oder ein pharmazeutisch annehmbares Salz davon durch intravenöse Infusion verabreicht wird.

**9.** Kit umfassend eine Verbindung der Formel:

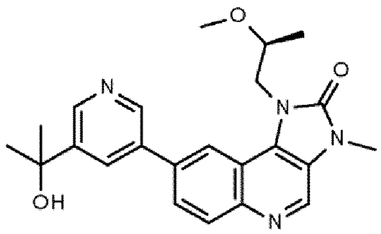

oder ein pharmazeutisch annehmbares Salz davon und eine Verbindung der Formel:

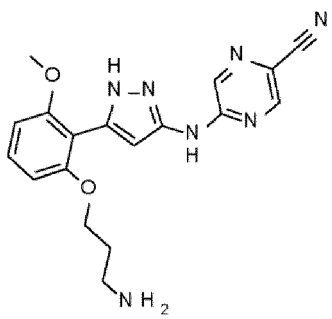

oder ein pharmazeutisch annehmbares Salz davon zur gleichzeitigen, getrennten oder aufeinanderfolgenden Verwendung bei der Behandlung von Krebsarten mit Plattenepithel-Histologie, Brustkrebs, Prostatakrebs, Blasenkrebs, Gebärmutterhalskrebs, Endometriumkrebs, Eierstockkrebs und kolorektalem Karzinom.

**10.** Kit zur Verwendung nach Anspruch 9, wobei die Krebsarten mit Plattenepithel-Histologie und der Brustkrebs Plattenepithel-NSCLC, HNSCC, Speiseröhrenkrebs, Analkrebs und TNBC sind.

**11.** Kit zur Verwendung nach Anspruch 10, wobei der Brustkrebs TNBC ist.

**12.** Kit umfassend eine pharmazeutische Zusammensetzung, die eine Verbindung der Formel:

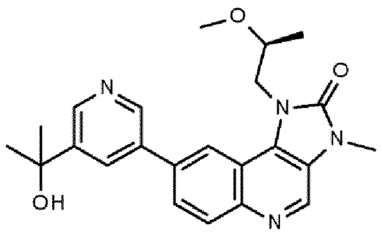

oder ein pharmazeutisch annehmbares Salz davon mit einem oder mehreren pharmazeutisch annehmbaren Trägern, Verdünnungsstoffen oder Hilfsstoffen umfasst, und eine pharmazeutische Zusammensetzung, die eine Verbindung der Formel:

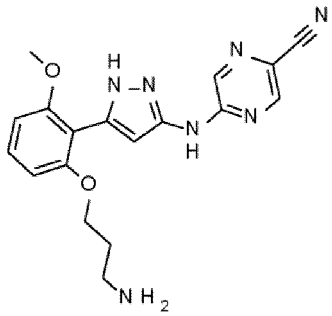

oder ein pharmazeutisch annehmbares Salz davon mit einem oder mehreren pharmazeutisch annehmbaren Trägern, Verdünnungsstoffen oder Hilfsstoffen umfasst, zur gleichzeitigen, getrennten oder aufeinanderfolgenden Verwendung bei der Behandlung von Krebsarten mit Plattenepithel-Histologie, Brustkrebs, Prostatakrebs, Endometriumkrebs, Eierstockkrebs und kolorektalem Karzinom.

**13.** Kit zur Verwendung nach Anspruch 12, wobei die Krebsarten mit Plattenepithel-Histologie und der Brustkrebs Plattenepithel-NSCLC, HNSCC, Speiseröhrenkrebs, Analkrebs und TNBC sind.

**14.** Kit zur Verwendung nach Anspruch 13, wobei der Brustkrebs TNBC ist.

## Revendications

**1.** Composé de la formule :

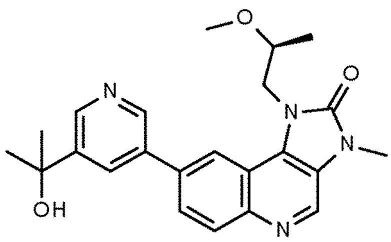

ou sel pharmaceutiquement acceptable de celui-ci, pour une utilisation simultanée, séparée, ou séquentielle en combinaison avec un composé de la formule :

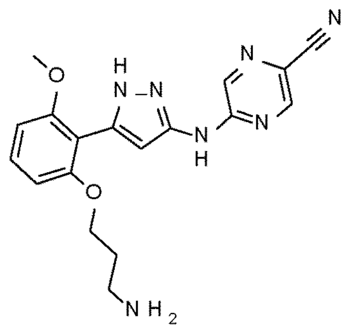

,

ou un sel pharmaceutiquement acceptable de celui-ci, dans le traitement de cancers histologiques squameux, d'un cancer du sein, d'un cancer de la prostate, d'un cancer de la vessie, d'un cancer du col de l'utérus, d'un cancer de l'endomètre, d'un cancer de l'ovaire, et d'un cancer colorectal.

**2.** Composé pour une utilisation selon la revendication 1, dans lequel les cancers histologiques squameux et le cancer du sein sont un cancer du poumon non à petites cellules (NSCLC) squameux, un cancer de la tête et du cou à cellules squameuses (HNSCC), un cancer de l'oesophage, un cancer de l'anus, et un cancer du sein triple négatif (TNBC).

**3.** Composé pour une utilisation selon la revendication 2, dans lequel le cancer du sein est un cancer du sein triple négatif (TNBC).

**4.** Composé pour une utilisation selon la revendication 1 dans lequel

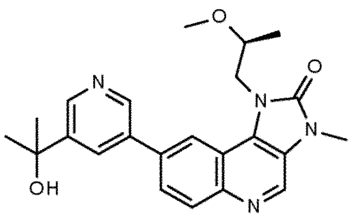

,

ou un sel pharmaceutiquement acceptable de celui-ci, est administré à une dose allant d'environ 100 mg deux fois par jour à environ 200 mg deux fois par jour les Jours 1 à 14 d'un cycle de 14 jours et

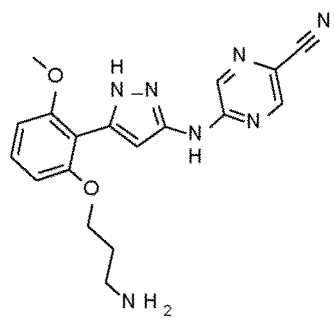

,

ou un sel pharmaceutiquement acceptable de celui-ci, est administré à une dose allant d'environ 60 mg/m$^2$ à environ 105 mg/m$^2$ le Jour 1 d'un cycle de 14 jours ou tous les 14 ou 21 jours.

**5.** Composé pour une utilisation selon la revendication 4 dans lequel

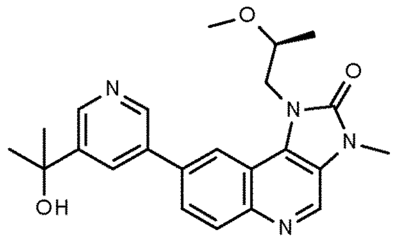

,

ou un sel pharmaceutiquement acceptable de celui-ci, est administré à une dose d'environ 100 mg deux fois par jour les Jours 1 à 14 d'un cycle de 14 jours et

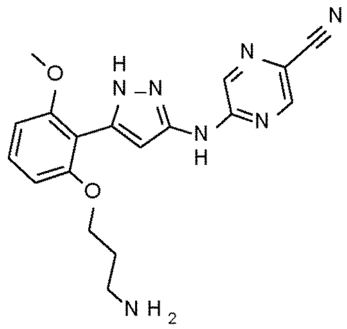

,

ou un sel pharmaceutiquement acceptable de celui-ci, est administré à une dose d'environ 60 mg/$m^2$ le Jour 1 d'un cycle de 14 jours ou tous les 14 ou 21 jours.

**6.** Composé pour une utilisation selon la revendication 4 dans lequel

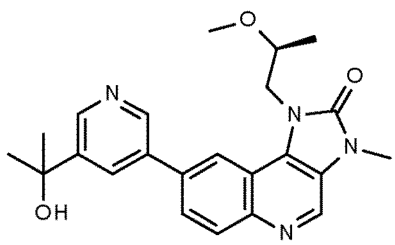

,

ou un sel pharmaceutiquement acceptable de celui-ci, est administré à une dose d'environ 100 mg deux fois par jour les Jours 1 à 14 d'un cycle de 14 jours et

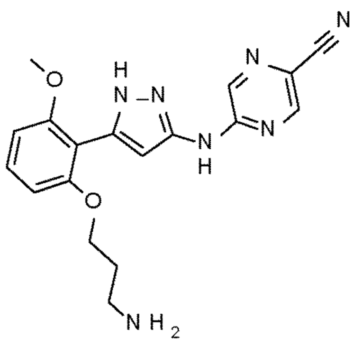

,

ou un sel pharmaceutiquement acceptable de celui-ci, est administré à une dose d'environ 105 mg/$m^2$ le Jour 1

d'un cycle de 14 jours ou tous les 14 ou 21 jours.

**7.** Composé pour une utilisation selon la revendication 4 dans lequel

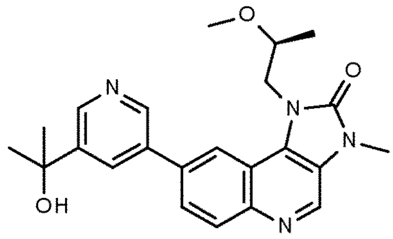

,

ou un sel pharmaceutiquement acceptable de celui-ci, est administré à une dose d'environ 200 mg deux fois par jour les Jours 1 à 14 d'un cycle de 14 jours et

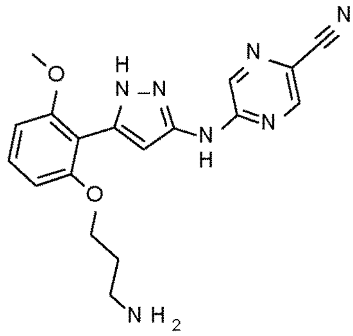

,

ou un sel pharmaceutiquement acceptable de celui-ci, est administré à une dose d'environ 105 mg/$m^2$ le Jour 1 d'un cycle de 14 jours ou tous les 14 ou 21 jours.

**8.** Composé pour une utilisation selon les revendications 4 à 7 dans lequel

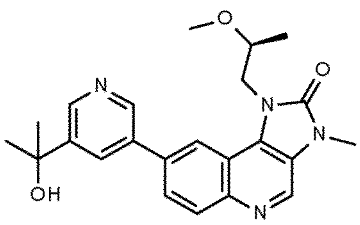

,

ou un sel pharmaceutiquement acceptable de celui-ci, est administré oralement et

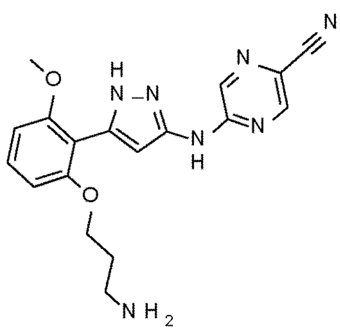

,

ou un sel pharmaceutiquement acceptable de celui-ci, est administré par infusion intraveineuse.

**9.** Kit comprenant un composé de la formule :

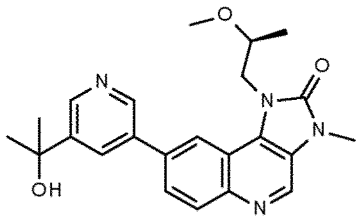

,

ou un sel pharmaceutiquement acceptable de celui-ci, et un composé de la formule :

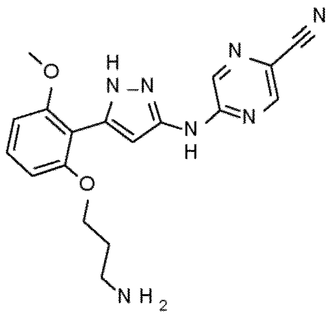

,

ou un sel pharmaceutiquement acceptable de celui-ci, pour une utilisation simultanée, séparée, ou séquentielle dans le traitement de cancers histologiques squameux, d'un cancer du sein, d'un cancer de la prostate, d'un cancer de la vessie, d'un cancer du col de l'utérus, d'un cancer de l'endomètre, d'un cancer de l'ovaire, et d'un cancer colorectal.

**10.** Kit pour une utilisation selon la revendication 9, dans lequel les cancers histologiques squameux et le cancer du sein sont un cancer du poumon non à petites cellules (NSCLC) squameux, un cancer de la tête et du cou à cellules squameuses (HNSCC), un cancer de l'oesophage, un cancer de l'anus, et un cancer du sein triple négatif (TNBC).

**11.** Kit pour une utilisation selon la revendication 10, dans lequel le cancer du sein est un cancer du sein triple négatif (TNBC).

**12.** Kit, comprenant une composition pharmaceutique, comprenant un composé de la formule :

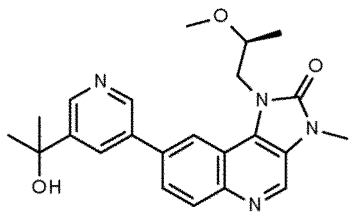

,

ou un sel pharmaceutiquement acceptable de celui-ci, avec un ou plusieurs véhicules, diluants, ou excipients pharmaceutiquement acceptables, et une composition pharmaceutique, comprenant un composé de la formule :

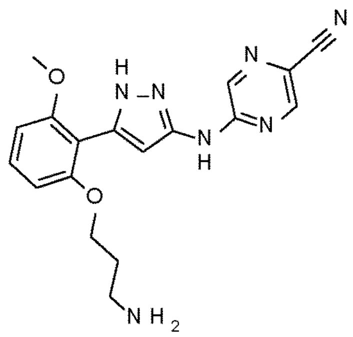

,

ou un sel pharmaceutiquement acceptable de celui-ci, avec un ou plusieurs véhicules, diluants, ou excipients pharmaceutiquement acceptables pour une utilisation simultanée, séparée, ou séquentielle dans le traitement de cancers histologiques squameux, d'un cancer du sein, d'un cancer de la prostate, d'un cancer de l'endomètre, d'un cancer de l'ovaire, et d'un cancer colorectal.

**13.** Kit pour une utilisation selon la revendication 12, dans lequel les cancers histologiques squameux et le cancer du sein sont un cancer du poumon non à petites cellules (NSCLC) squameux, un cancer de la tête et du cou à cellules squameuses (HNSCC), un cancer de l'oesophage, un cancer de l'anus, et un cancer du sein triple négatif (TNBC).

**14.** Kit pour une utilisation selon la revendication 13, dans lequel le cancer du sein est un cancer du sein triple négatif (TNBC).

FIG. 1/3

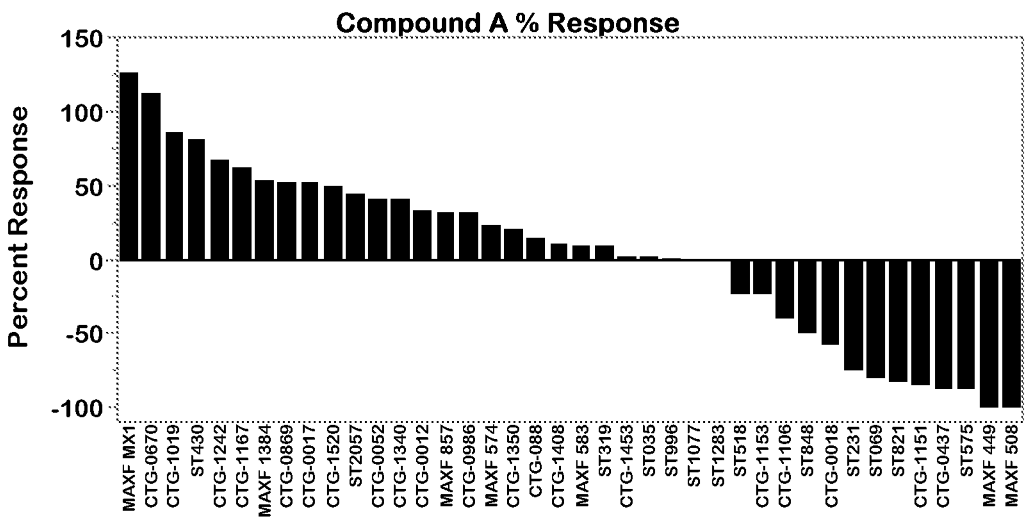
Compound A % Response
Percent Response
150
100
50
0
-50
-100
MAXF MX1
CTG-0670
CTG-1019
ST430
CTG-1242
CTG-1167
MAXF 1384
CTG-0869
CTG-0017
CTG-1520
ST2057
CTG-0052
CTG-1340
CTG-0012
MAXF 857
CTG-0986
MAXF 574
CTG-1350
CTG-088
CTG-1408
MAXF 583
ST319
CTG-1453
ST035
ST996
ST1077
ST1283
ST518
CTG-1153
CTG-1106
ST848
CTG-0018
ST231
ST069
ST821
CTG-1151
CTG-0437
ST575
MAXF 449
MAXF 508

# FIG 2/3

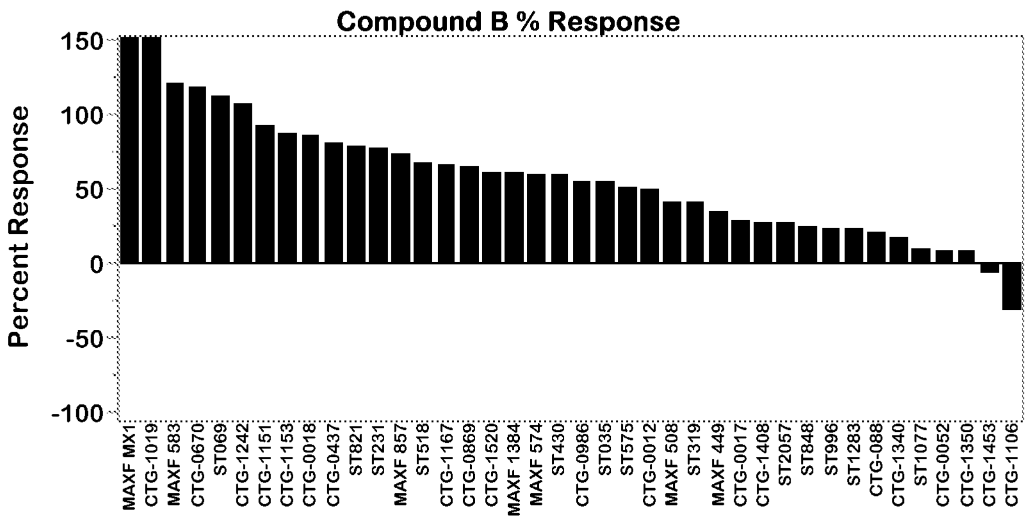
Compound B % Response
Percent Response
150
100
50
0
-50
-100
MAXF MX1
CTG-1019
MAXF 583
CTG-0670
ST069
CTG-1242
CTG-1151
CTG-1153
CTG-0018
CTG-0437
ST821
ST231
MAXF 857
ST518
CTG-1167
CTG-0869
CTG-1520
MAXF 1384
MAXF 574
ST430
CTG-0986
ST035
ST575
CTG-0012
MAXF 508
ST319
MAXF 449
CTG-0017
CTG-1408
ST2057
ST848
ST996
ST1283
CTG-088
CTG-1340
ST1077
CTG-0052
CTG-1350
CTG-1453
CTG-1106

FIG 3/3

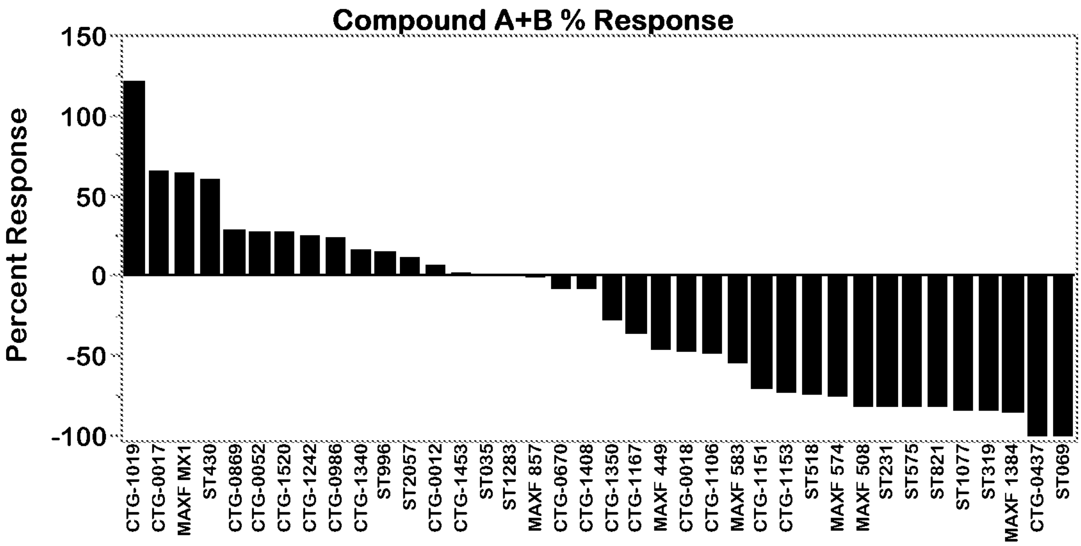
Compound A+B % Response
Percent Response
150
100
50
0
-50
-100
CTG-1019
CTG-0017
MAXF MX1
ST430
CTG-0869
CTG-0052
CTG-1520
CTG-1242
CTG-0986
CTG-1340
ST996
ST2057
CTG-0012
CTG-1453
ST035
ST1283
MAXF 857
CTG-0670
CTG-1408
CTG-1350
CTG-1167
MAXF 449
CTG-0018
CTG-1106
MAXF 583
CTG-1151
CTG-1153
ST518
MAXF 574
MAXF 508
ST231
ST575
ST821
ST1077
ST319
MAXF 1384
CTG-0437
ST069

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012097039 A
- WO 2010077758 A
- WO 2013103836 A


### Non-patent literature cited in the description


- **JUVEKAR et al.** *Cancer Discov.,* 2012, vol. 2 (11), 1048-1063
- **KUMAR et al.** *PNAS USA,* 2011, vol. 107, 7491-7496
- **IBRAHIM et al.** *Cancer Discov.,* 2012, vol. 2 (11), 1036-1047
- **SHEN et al.** *Cancer Res.,* 2012, vol. 72 (8
- **MASSEY et al.** *Molecular Oncology,* 2015, 1-12
- *CHEMICAL ABSTRACTS,* 1386874-06-1
- *CHEMICAL ABSTRACTS,* 1234015-52-1
- *CHEMICAL ABSTRACTS,* 1234015-57-6
- Characterization of crystalline and partially crystalline solids by X-ray powder diffraction (XRPD). The U. S. Pharmacopeia 35 - National Formulary 30. 01 December 2012
- **P. STAHL et al.** HANDBOOK OF PHARMACEUTICAL SALTS: PROPERTIES, SELECTION AND USE. VCHA/Wiley-VCH, 2002
- **L.D. BIGHLEY ; S.M. BERGE ; D.C. MONKHOUSE.** Encyclopedia of Pharmaceutical Technology. Marcel Dekker, Inc, 1995, vol. 13, 453-499
- **S.M. BERGE et al.** Pharmaceutical Salts. *Journal of Pharmaceutical Sciences,* January 1977, vol. 66 (1
- Remington: The Science and Practice of Pharmacy. Pharmaceutical Press, 2012
- **BLISS, C. I.** *Ann Appl Biol,* 1939, vol. 26 (3), 585-615
- **NEUENSCHWANDER et al.** *Statist. Medicine,* 2008, vol. 27, 2420-2439
- **NEUENSCHWANDER et al.** *Statist. Medicine,* 2008
- **EISENHAUER et al.** *EJC,* 2009, vol. 45, 228-247